(19) Europäisches Patentamt
European Patent Office
Office européen des brevets

(11) EP 3 907 293 B1

(12) **EUROPEAN PATENT SPECIFICATION**

(45) Date of publication and mention of the grant of the patent:
**13.08.2025 Bulletin 2025/33**

(21) Application number: **19896991.7**

(22) Date of filing: **11.12.2019**

(51) International Patent Classification (IPC):
***C12Q 1/68*** *(2018.01)* ***G01N 33/543*** *(2006.01)*

(52) Cooperative Patent Classification (CPC):
**G01N 33/54353; B01J 19/0046;** B01J 2219/00529; B01J 2219/00549; B01J 2219/00608; B01J 2219/00722; B01J 2219/00725

(86) International application number:
**PCT/CN2019/124473**

(87) International publication number:
**WO 2020/119706 (18.06.2020 Gazette 2020/25)**

(54) **BIOCHIP AND MANUFACTURING METHOD AND APPLICATION THEREOF**

BIOCHIP UND HERSTELLUNGSVERFAHREN SOWIE VERWENDUNG DESSELBEN

BIOPUCE, PROCÉDÉ DE FABRICATION ET APPLICATION CORRESPONDANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2018 CN 201811514682**

(43) Date of publication of application:
**10.11.2021 Bulletin 2021/45**

(73) Proprietors:
- **BGI Shenzhen**
  **Shenzhen, Guangdong 518083 (CN)**
- **BGI Shenzhen Co., Limited**
  **Guangdong 518083 (CN)**

(72) Inventors:
- **WANG, Jian**
  **Shenzhen, Guangdong 518083 (CN)**
- **XU, Xun**
  **Shenzhen, Guangdong 518083 (CN)**
- **WANG, Jun**
  **San Diego, California 92130 (US)**
- **SHEN, Yue**
  **Shenzhen, Guangdong 518083 (CN)**
- **NI, Ming**
  **Shenzhen, Guangdong 518083 (CN)**
- **ZHANG, Wenwei**
  **Shenzhen, Guangdong 518083 (CN)**
- **WANG, Yong**
  **Shenzhen, Guangdong 518083 (CN)**
- **JIANG, Xianger**
  **Shenzhen, Guangdong 518083 (CN)**
- **HUANG, Xiaoluo**
  **Shenzhen, Guangdong 518083 (CN)**

(74) Representative: **Lavoix**
**Bayerstraße 83**
**80335 München (DE)**

(56) References cited:

| | |
|---|---|
| **WO-A1-2017/127570** | **WO-A1-2017/169196** |
| **WO-A2-02/096552** | **CN-A- 103 645 308** |
| **CN-A- 106 311 365** | **CN-A- 106 872 562** |
| **CN-A- 107 475 071** | **US-A1- 2003 203 369** |
| **US-A1- 2004 152 081** | **US-A1- 2007 218 484** |
| **US-A1- 2013 196 880** | **US-A1- 2016 265 032** |

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 907 293 B1

## Description

### Technical field

The present application relates to the field of biochemistry. Particularly, the present application relates to a biochip, method of preparation and use thereof.

### Background

Biomacromolecules mainly include DNA, RNA, polypeptides, polysaccharides and so on. Due to important uses of these biomacromolecules in the fields of medicines, agricultures, food, materials, environment and the like, in-vitro synthesis of these biomacromolecules has become a hotspot for researches, and has a huge market prospect. Since the 1950s, Todd and Khorana research groups reported DNA synthesis (Michelwn, A. M., Todd, A. R. J. Chem. Soc., 1955; Gilham, P. T., Khorana, H. G., J. A. m. Cliem. Soc., 1958) for the first time, methods of DNA synthesis have been developed for a long term. At present, classical synthesis methods include column type synthesis method developed in the 1980s, and microarray-based high-throughput synthesis method developed in the 1990s. These methods are basically solid-phase synthesis methods, in which a single deoxyribonucleotide is used as a unit for synthesis, and the most of synthesis processes involve a four-step cycle based on a phosphoramidite chemical method: deprotection, coupling, capping and oxidation steps. Due to the incompleteness of the reaction in each step, accompanying possible side effects (such as deadenylation) and the decrease in reactant concentration with the reaction progress, with the prolong of the DNA single strand, the error rate of DNA synthesis sharply increased and the yield dramatically decreased. In addition, the disadvantage of the column type synthesis method has the disadvantages of large amount of reagent used and low throughput, which causes facts that the synthesis is high in cost and consuming in time and labor. Although the microarray-based synthesis method has high throughput and small amount of reagent used, it has relatively high error rate, low yield and low stability. The synthesis methods of other biomacromolecules, such as RNA, polypeptides and polyphosphoric acids, are similar to the solid-phase synthesis method of DNA, in which single-cycle chemical reaction is repeated on the solid-phase carrier; and their synthesis characteristics are also similar. The column type synthesis method has a low error rate, but has large reagent consumption amount and low throughput, which is not conducive to cost saving. The microarray-based synthesis method has high throughput and small material amount, but relatively high error rate and low stability. Therefore, it is extremely important to find a synthesis chip which will be capable of achieving high throughput, low cost and low error rate.

Biochips are also referred to as protein chips or gene chips, which are originated from a combination of DNA hybridization probe technique and semiconductor industrial technique. This technique is that a large number of probe molecules are fixed on a support and then hybridized to DNA or other sample molecules (for example, proteins, factors or small molecules) with fluorescent marks, and the quantity and sequence information of the sample molecule are obtained by detecting the hybridization signal intensity of each probe molecule. The biochip technique is originated from hybridization of nucleic acid molecules. The so-called biochip generally refers to a microarray hybridized chip (micro-arrays), in which both the sequence and location of each molecule in the array have been known, of a biological information molecule (for example, gene fragment, DNA fragment or polypeptide, protein, sugar molecule and tissue) fixed on a mutually support medium at high density, and is preset sequence point array. Biochemical analysis processes are integrated on the surfaces of the chips according to a specific interaction principle between biological molecules, thereby achieving high-throughput and rapid detection of DNA, RNA, polypeptide, protein and other biological components. The narrow concept of the biochip means a biomolecule point array formed by fixing biomolecules (oligonucleotides, cDNA, genomic DNA, polypeptides, antibodies, antigens, etc.) on a solid-phase matrix such as silicon wafer, glass sheet (bead), plastic sheet (bead), gel and nylon membrane through different methods. Therefore, the biochip technique is also referred to as a microarray technique. The solid-phase matrix containing lots of biological information is called a microarray, also referred to as a biochip. On the basis of such chips, the biochip has developed into microfluidic chip, also known as microelectronic chip, that is, micro-laboratory chip. Like the electronic chip, the biochip is also integrated, however, the integration on the biochip is integration of biomaterials. Similar to laboratory detection, the detection of blood glucose, protein, enzymatic activity and the like on the biochip is based on the same biological reaction principles. Thus, the biochip is a carrier platform. Since the biochip has potential advantages such as high integration degree, high throughout, high sensitivity and portable carrying, it is widely applied to many scenes, specifically in the aspects of biological synthesis and detection, and it has a huge application prospect.

At present, for the representative commercialized microarray synthesizer, such as CustomArray synthesizer, the synthesis reaction is shrunk into micron-level reaction holes, and there are tens of thousands of reaction holes on one chip. In this way, the synthesis throughput is improved and the consumption of raw materials is reduced to a certain extent, however, the yield is low, the electrochemical reaction is not easy to control, and the error rate is high. In addition, temperature and humidity sensor, control circuit and the like are also integrated on the chip, thereby the production process

is very complex, and the price of the chip is relatively expensive. For the Twist synthesizer, via using a high-speed microscale ink-jet printing head as a transmission manner of a reagent such as a monomer, Oligo is synthesized on a specially treated micron-level silicon-based chip's through-via, and then a matched reactor is used to dock with these micro-holes to realize in-situ PCR and PCA, so as to directly obtain a large number of long DNA molecule fragments. The biochip is made of a special silicon-based material with specific chemical modification. It has large throughput and needs physical isolation. In addition, the biochip has a large size (the size is equivalent to that of a conventional 96-well plate) and has not been commercialized. The synthesizer controlled by Evonetix circuit achieves independent control of reactions on different sites by mainly utilizing the characteristics of large-scale parallel control of a semiconductor. However, in practical application, an important problem is how to avoid the interaction of reactions on different sites and to control of product output. At the beginning of 2018, the British company Evonetix announced a synthesis technology based on semiconductor chip control. The key point of the Evonetix technology lies in the Oligo synthesis of theoretically nearly one billion sites and the high-fidelity DNA error correction assembly technology which can be monitored in real time. The principle of controlling the synthesis process is as follows: in the closed chamber of specially-designed, large-scale addressable synthesis sites, a blocking material (such as n-tetracosane) having a low melting point and capable of being repeatedly heated is added, the synthesizer uses a circuit signal to control whether each site is electrified or not, and then select whether to heat the site. In the case of heating, the special material on the site is adsorbed on the site to prevent the subsequent introduced reagent from reacting on the site. If the subsequent synthesis needs to be carried out at the site, the material will be washed away with a solvent to expose the site for synthesis reaction. In this way, the individual control of reaction on each site is achieved. However, in addition to the special chemical modification, circuit control is integrated on the chip, and therefore the fabrication process of the chip is complex. US 2007/218484, US 2004/152081, and US 2003/203369 disclose a biochip for polynucleotide synthesis and the corresponding method of manufacture, carrying a chemical entity (such as a linker molecule capable of initiating a DNA synthesis reaction) and a code (such as bar codes), the code having a unique corresponding relationship with the biochip.

Therefore, there is an urgent need for a new technological breakthrough in this field, especially a biochip having a simple manufacturing process, a low price and multi-detection throughput, reusable and having a recognition function.

## Summary

The application provides a biochip, a method for preparing the same and use thereof. The biochip of the application can be applied to synthesis and detection of biomacromolecules such as DNA, RNA and polypeptide, antibody screening, antigen recognition and the like, and has a huge application prospect. The biochip is according to claim 1, the method for preparing the biochip is according to claim 10, the use of the biochip is according to claim 13 and a method for synthesizing a nucleic acid is according to claim 14.

In an aspect, the present application provides a biochip, the biochip carrying a chemical entity and a code, wherein the code has a unique corresponding relationship with the chip, wherein the biochip has a surface treated with a silanizing reagent, and the silanizing reagent is a 50% silanizing reagent, and wherein the chemical entity is a linker molecule capable of initiating a DNA/RNA synthesis reaction, and the biochip is made of silicon wafer.

In another aspect, the present application provides a method for preparing the biochip of the application, and the method comprising the following steps:

1) coding the biochip;
2) pretreating the biochip;
3) performing silanization treatment on the surface of the biochip, wherein the silanization treatment is performed on the surface of the biochip by soaking the biochip in a silanizing reagent and carrying out ultrasonic treatment, and the silanizing reagent is a 50% silanizing reagent;
4) chemically modifying the surface of the biochip; and
5) optionally, detecting or quantitatively analyzing the chemical modification.

In another aspect, the present application provides use of the biochip of the application in synthesis of a nucleic acid.

## Brief description of the drawings

Fig. 1 schematically depicts an example of a recognizable 2-dimensional bar code chip according to the application.
Fig. 2 schematically depicts a process of DNA synthesis using a chip according to the application.
Fig. 3 schematically depicts a process of polypeptide synthesis using a chip according to the application.

Fig. 4A shows an HPLC chromatogram of a T30 product synthesized in experiment 1 in Example 6.
Fig. 4B shows an HPLC chromatogram of a T30 product synthesized in experiment 2 in Example 6.
Fig. 4C shows an HPLC chromatogram of a T30 product synthesized in experiment 3 in Example 6.
Fig. 4D shows an HPLC chromatogram of a T30 product synthesized in experiment 4 in Example 6.
Fig. 4E shows an HPLC chromatogram of a T30 product synthesized in experiment 5 in Example 6.
Fig. 4F shows an HPLC chromatogram of a standard sample of T30 in Example 6.
Fig. 5 shows a gel electrophoretogram of a product synthesized in Example 7, wherein, Ctrl: standard synthetic primer control; lanes 1-3: Mix1-3.

## Detailed description

In the present application, unless otherwise defined, all technical and scientific terms used herein have the meanings generally understood by those skilled in the art of the present application. In the embodiments of the present application, methods and materials similar to or equivalent to those described herein may be used, and illustrative and suitable methods and materials are described below. Furthermore, the materials, methods and embodiments are only exemplary and not restrictive. Meanwhile, in order to better understand the invention, the application also provides definitions and explanations of related terms. In case of any conflict between the definitions and explanations of the terms provided in this application and the meanings generally understood by ordinary technical personnel in the field of this application, the definitions and explanations of the terms provided in this application shall be prevailed.

### Biochip

In an aspect, the present application provides a biochip, the biochip carrying a chemical entity and a code, wherein the code has a unique corresponding relationship with the chip, wherein the biochip has a surface treated with a silanizing reagent, and the silanizing reagent is a 50% silanizing reagent, and wherein the chemical entity is a linker molecule capable of initiating a DNA/RNA synthesis reaction, and the biochip is made of silicon wafer.

The chips may have any desired shapes. For example, the chip may be in a form of flake, cuboid, cylinder, sphere and the like, preferably, the chip is in a form of square flake. In a specific embodiment, the biochip is a microchip for DNA synthesis, which has a size of 2 mm*2 mm, even 1 mm*1 mm, 0.5 mm*0.5 mm or less. In some preferred embodiments, the chip of the application is reused.

### Code

The chip of the application has recognizable characteristics. For this purpose, it is needed to code the chip, which provides a specific signal for the chip. The specific signal on the chip may be a magnetic signal, an electrical signal, an identification code and the like, preferably, the specific signal is a 2-dimensional bar code.

Therefore, in a specific embodiment, the biochip of the application carries a code, and the code has a unique corresponding relationship with the chip. It should be understood that the "code" herein means any characteristic used for distinguishing and identifying the identity of the chip. The characteristic includes but is not limited to a number, symbol, graph, identification code such as bar code and 2-dimensional bar code, preferably 2-dimensional bar code.

In a preferred embodiment, the code carried on the chip is a 2-dimensional bar code. The 2-dimensional bar code is used for recording data symbol information using black and white graph formed by distributing certain specific geometric graph on a plane (in 2-dimensional direction) according to a certain rule; word or numerical value information is represented by using several geometrical shapes corresponding to a binary system by cleverly utilizing the concept of bit streams "0" and "1" constituting an internal logic basis of a computer on a code system, and automatic information processing is achieved through automatic reading via an image input equipment or photoelectric scanning equipment. It has some generalities of the bar code technique: each code system has its specific character set; each character occupies a certain width; and it has a certain verification function, and the like. Meanwhile, it also has functions of automatically recognizing information in different lines and processing graph rotation change points. The 2-dimensinal bar code has a built-in error correction function, can restore data in the case that the code is damaged or smeared. It can adopt mathematical error correction (Reed-Solomon) to restore data.

In some preferred embodiments, the code carried on the chip is a combination of at least two or more features. For example, the code carried on the chip may be a combination of an identification code (for example, a bar code or a 2-dimensional bar code) and an electrical signal, a combination of an identification code (for example, a bar code or a 2-dimensional bar code) and an light signal (for example, fluorescence), a combination of an identification code (for example, a bar code or a 2-dimensional bar code) and a number, a combination of an identification code (for example, a bar code or a 2-dimensional bar code) and an RFID tag, a combination of an RFID tag and an electrical signal, a combination of an RFID lab and a light signal (for example fluorescence), a combination of an RFID lab and a number, a combination of a number

and a light signal (for example, fluorescence), or a combination of a number, a graph and an identification code (for example, a bar code or a 2-dimensional bar code), etc.

One important function of the code is to distinguish and identify the chip. The expression "the code has a unique corresponding relationship with the chip" means that each chip corresponds to one unique code. In other words, the codes carried on chips are different from one another. Since each chip has a unique corresponding relationship with the carried code, by recognition of the codes, a plurality of chips can be conveniently distinguished, or the chip of the interest can be rapidly and conveniently sought and identified from the plurality of chips.

In the method of the application, the codes carried on the chips are recognized in various manners. In some preferred embodiments, the codes carried on the chip are detected by using a detector (e.g., detector by recognizing a number, a symbol, graph, an identification code (e.g., a bar code, a 2-dimensional bar code), an RFID tag, a light signal (e.g., fluorescence, chemiluminescence, Raman spectroscopy), a quantum dot, a magnetic signal, an electrical signal, or any combination thereof), and the signal detected by the detector is analyzed by using a processor, thereby recognizing the identity (code) of the chip. Various methods and instruments for detecting/recognizing the number, symbol, graph, bar code, 2-dimensional bar code, RFID tag, fluorescence, luminescence, quantum dot, Raman spectroscopy and the like are known by those skilled in the art, including but not limited to an light signal recognizer, a magnetic signal recognizer, an electrical signal recognizer, an image recognizer or any combination thereof. For example, the instrument is a 2-dimensional bar code detector, a bar code detector or an RFID tag reader, etc.

Chemical modification on the surface of the chip

After the code is added on the chip, chemical modification is performed on the surface of the chip to achieve the purpose that the chip meets different functional requirements. Specific types of molecules modified on the surface of the chip depend on practical applications of the chip. For example, the surface of the chip used for synthesis of a biomacromolecule is modified with a molecule capable of initiating a synthesis reaction; the surface of the chip used for detection of an antigen is modified with a specific antibody; the surface of the chip used for screening of an antibody is modified with a specific antigen.

A. For synthesis of biomacromolecules

In order to synthesize different varieties of biomacromolecules such as DNA, RNA and polypeptides on the surface of the chip, it is needed to chemically modify the corresponding surface of each chip; in addition, it is also needed to ensure there are enough modification molecules on the surface of the chip while guaranteeing that the molecules modified on the surface of the chip are homogeneously distributed, so as to avoid inhomogeneous signal distribution.

For this purpose, in a specific aspect, the present application provides a biochip, the biochip carrying a chemical entity and a code, wherein the chemical entity reacts with a reaction reagent so as to link a monomer in the reaction reagent to the terminal of the chemical entity, and the code has a unique corresponding relationship with the biochip, wherein the biochip has a surface treated with a silanizing reagent, and the silanizing reagent is a 50% silanizing reagent; and wherein the chemical entity is a linker molecule capable of initiating a DNA/RNA synthesis reaction, and the biochip is made of silicon wafer.

As used herein, the biochip of the application is chemically modified, namely, the surface of the biochip is modified by linking the chemical entity. The chemical entity is a linker molecule (connection molecule), which reacts with a reaction reagent (coupling reaction or linking reaction), thereby linking a monomer in the reaction reagent to the terminal of the linker molecule.

The biochip for synthesis of nucleic acids (DNA/RNA) is modified with the linker molecule, wherein the linker molecule is preferably a compound having a functional group reacting with an amino group on the beginning end, hydroxyl with an acid-labile protecting group on the terminal, wherein the protecting group is removed and the hydroxyl is exposed upon treating with acid, and any one or more functional groups selected from ester group, lipid group, thioester group, o-nitrobenzyl group, coumarin group, hydroxyl, mercapto, mercapto ether group, carboxyl, formyl, amino, amido, amide group, alkenyl and alkynyl in the middle, preferably, the functional group at the beginning end is carboxyl, and the hydroxyl protecting group at the terminal is triphenylmethyl, for example 4,4'-dimethoxytriphenylmethyl (DMT), which will be removed with a solution of dichloroacetic acid, trichloroacetic acid or trifluoroacetic acid in an organic solvent (such as dichloromethane and acetonitrile). In a preferred embodiment, the Linker molecule is a molecule which will be broken and easily released off upon ammonolysis after DNA synthesis is completed, the preferred Linker molecule is Universal Linker, wherein the Universal Linker refers to exo-N-phenyl-5-(succinyloxy)-6-dimethyl-7-oxabicyclo[2.2.1]heptane-2-3-dicarbimide, exo-N-methyl-5-(succinyloxy)-6-dimethyl-7-oxabicyclo[2.2.1]heptane-2-3-dicarbimide or exo-N-methyl-5-(diglycoloxy)-6-dimethyl-7-oxabicyclo[2.2.1]heptane-2-3-dicarbimide. The chemical structure formula of Universal Linker is as follows:

(1) exo-N-phenyl-5-(succinyloxy)-6-dimethyl-7-oxabicyclo[2.2.1]heptane-2-3-dicarbimide

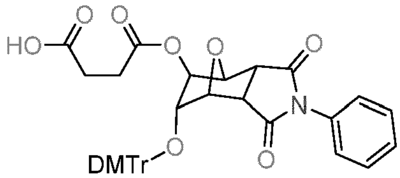

(2) exo-N-methyl-5-(succinyloxy)-6-dimethyl-7-oxabicyclo[2.2.1]heptane-2-3-dicarbimide

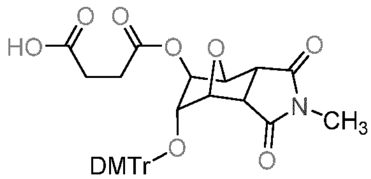

(3) exo-N-methyl-5-(diglycoloxy)-6-dimethyl-7-oxabicyclo[2.2.1]heptane-2-3-dicarbimide

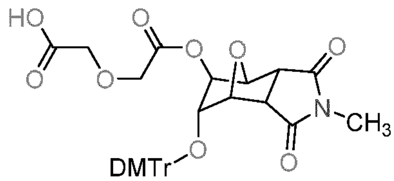

The biochip for synthesis of polypeptides is modified with a chemical entity, wherein the chemical entity is a polymer carrier (resin) for synthesis of polypeptides with a solid phase method. The resin is directly linked to a first amino acid (initial amino acid) only when a reaction group is introduced. According to the introduced reaction group, the resins and resin derivatives are divided into chloromethyl resin, carboxyl resin, amino resin or hydrazide resin. The chloromethyl resin such as Merrifield resin is generally selected for BOC synthesis method; carboxyl resin such as Wang resin and 2-Cl(Trt)-Clresin are generally selected for the FMOC synthesis method. The immobilization of the initial amino acid is mainly achieved by a covalent bond formed between the carboxyl protecting the amino acid and the reaction group of the resin. There are many ways to form the covalent bond: for chloromethyl resin, generally tetramethyl ammonium salt or sodium salt, potassium salt or cesium salt which protects amino acid is first prepared, and then the product directly reacts with the resin or reacts in an appropriate organic solvent such as dioxane, DMF or DMSO at an appropriate temperature; for carboxyl resin, an appropriate condensing agent such as DCC or carboxyl diimidazole is generally added so that the protected amino acid and the resin form co-ester to complete the immobilization of amino acid; for amino resin or hydrazide resin, the immobilization of amino acid is completed through an amido bond formed between the protected amino acid and the resin, after addition of an appropriate condensation agent such as DCC.

In the preferred technical solution of the biochip for synthesis of polypeptides, the chemical entity (linker molecule) for modifying the chip is the carboxyl resin, which is a molecule having a group capable of condensing with carboxyl at the terminal, such as Wang resin and 2-Cl(Trt)-Cl resin, or a molecule having a similar chemical structure; preferably, a linker molecule which has a functional group capable of reacting with an amino on the beginning end and a functional group capable of coupling with carboxyl of an amino acid monomer on the terminal, and is capable of being dissociated when in acid treatment, a compound having any one or more functional groups of ester group, lipid group, thioester group, o-nitrobenzyl group, coumarin group, hydroxyl, mercapto, mercapto ether group, carboxyl, formyl, amino, amido, amide group, alkenyl and alkynyl in the middle, such as 4-hydroxymethyl benzoic acid, 4-chloromethyl benzoic acid, or substituted 4-hydroxymethyl benzoic acid or 4-chlorohydroxymethyl benzoic acid, and the preferred linker molecule is 4-hydroxymethyl benzoic acid.

B. For biodetection

In another specific aspect, the present application provides a biochip, the biochip carrying a specific antigen/antibody and a code, and the code has a unique corresponding relationship with the chip. For example, the surface of the chip for antigen detection is modified with the specific antibody; the surface of the chip for antibody screening is modified with the specific antigen.

Preparation of biochips

In another aspect, the application provides a method for preparing a biochip, the method comprising the following steps:

1) coding the chip;
2) pretreating the chip;
3) carrying out silanization treatment on the surface of the chip, wherein the silanization treatment is performed on the surface of the biochip by soaking the biochip in a silanizing reagent and carrying out ultrasonic treatment, and the silanizing reagent is a 50% silanizing reagent;
4) chemically modifying the surface of the chip;
5) optionally, detecting or quantitatively analyzing the chemical modification.

As used in the invention, "coding the chip" means adding a unique identify tag for the chip. It should be understood that "code" here means any features for distinguishing and identifying the identity of the chip. The features include but are not limited to numbers, symbols, graphs, identification codes such as bar codes and 2-dimensional bar codes. In a preferred embodiment, "coding the chip" includes adding a 2-dimensional bar code on the chip, the 2-dimensional bar code being uniquely corresponding to the chip. In other words, the 2-dimensional bar codes added on different chips are different from one another.

In a specific embodiment, according to the invention, the 2-dimensional bar code is printed on the chip using laser printing, as a recognizable signal. To adapt to different application scenes, it is needed to design and fabricate chips that have different types of polishing and 2-dimensional bar code, namely, a non-polished single-sided 2-dimensional bar code chip, a non-polished double-sided 2-dimensional bar code chip, a single-polished single-sided 2-dimensional bar code chip, a single-polished double-sided 2-dimensional bar code chip, a double-polished single-sided 2-dimensional bar code chip and a double-polished double-sided 2-dimensional bar code chip. The surface of the chip is specially treated in the application scenes with special requirements, such as frosting or nano point location modification to increase surface area and ensure the rapid, accurate and efficient recognition of the 2-dimensional bar code chip.

Taking a 2-dimensional bar code chip for DNA synthesis as an example, the following technology is adopted: the size of the chip for DNA synthesis proposed by the application may be adjusted according to the application situation, for example, 2 mm * 2 mm, or even 1 mm * 1 mm, 0.5 mm * 0.5 mm or less, and the minimum may be sub-millimeter level (see Fig. 1 for example). Because the size of the chip is small, the dot 2-dimensional bar code mode is selected as the dot mode. In order to ensure the cutting quality and no burr, the 2-dimensional bar code chip proposed in the application is mechanically cut. In order to ensure the coding depth, the red laser is selected so that the point location is deep and clear, so even if there are some abrasions in the subsequent long-term use, the recognition of the 2-dimensional bar code is still not affected. To solve the problem that a large number of chips are inevitably adhered in the process of use, an oscillating reaction container is used to ensure the high efficiency in the process of use without affecting the uniformity of chip reaction on the surface of the chip. Recognition of chips is a photographing, enlarging and recognizing process of chips. In order to better recognize the chip, the points in the 2-dimensional bar code are as large and deep as possible.

In a specific embodiment, a plurality of 2-dimensional bar codes different from one another are printed on the whole silicon wafer at equal intervals, for example the 2-dimensional bar code may be 1 mm * 1 mm in size. Then, the silicon wafer is cut into 2 mm*2mm chips, and it is ensured that the 2-dimensional bar code is located in the center of each chip when in cutting.

As used herein, "pretreating the chip" is to achieve the purpose of cleaning the surface of the chip and meanwhile exposing more hydroxyl to improve the activity of surface reaction. The pretreatment steps include acid treatment, alkali treatment, ultrasonic treatment, plasma cleaning, acetone washing or a combination thereof, wherein the acid is selected from sulfuric acid, hydrochloric acid, phosphoric acid, piranha liquid and the like, the alkali may be selected from sodium hydroxide and potassium hydroxide and the like. In a preferred embodiment, the pretreatment method is acid treatment followed by plasma cleaning; more preferably, the pretreatment method is sodium hydroxide treatment followed by plasma cleaning and acetone washing.

As used herein, "carrying out silanization treatment on the surface of the chip" refers to carrying out amino modification on the surface of the chip. In the process of silanization, one or more of a chemical vapor deposition (CVD) method, solution soaking, simple negative pressure vapor deposition method and the like are adopted, wherein the silanizing reagent is a reagent in which an alkoxy silicon group is at one end and amino is at the other end, and may be selected from APTMS, APTES and the like, preferably, APTMS is used as the silanizing reagent, further wherein, a 50% silanizing reagent is used. In a preferred embodiment, the simple negative pressure vapor deposition method is adopted so as to ensure convenient operation and uniform surface modification while ensuring efficient silanization.

As used herein, "chemically modifying the surface of the chip" means linking the chemical entity onto the surface of the chip. The specific varieties of chemical entities depend on specific applications of the chip.

When the biochip is used for synthesis of nucleic acids (DNA/RNA), the chemical entity is preferably a linker molecule having a functional group capable of reacting with amino at the beginning end and hydroxyl with an acid-labile protecting group at the terminal, and the protecting group is removed to expose the hydroxyl when in acid treatment, and having any one or more functional groups of ester group, lipid group, thioester group, o-nitrobenzyl group, coumarin group, hydroxyl, mercapto, mercapto ether group, carboxyl, formyl, amino, amido, amide group, alkenyl and alkynyl in the middle, preferably the beginning end is carboxyl, the protecting group at the terminal is triphenylmethyl, for example 4,4'-dimethyoxytripheylmethyl (DMT), which will be removed when in treatment with solutions of dichloroacetic acid, trichloroacetic acid or trifluoroacetic acid in organic solvents (such as dichloromethane and acetonitrile).

When the biochip is used for synthesis of polypeptide, the linker molecule is preferably carboxyl resin, namely, a molecule having a group capable of condensing with carboxyl at the terminal, such as Wang resin and 2-Cl(Trt)-Cl resin, or a molecule having a similar chemical structure; preferably, a linker molecule which has a functional group capable of reacting with an amino on the beginning end and a functional group capable of coupling with carboxyl of an amino acid monomer on the terminal, and is capable of being dissociated when in acid treatment, a compound having any one or more functional groups of ester group, lipid group, thioester group, o-nitrobenzyl group, coumarin group, hydroxyl, mercapto, mercapto ether group, carboxyl, formyl, amino, amido, amide group, alkenyl and alkynyl in the middle, such as 4-hydroxymethyl benzoic acid, 4-chloromethyl benzoic acid, or substituted 4-hydroxymethyl benzoic acid or 4-chlorohydroxymethyl benzoic acid, and the preferred linker molecule is 4-hydroxymethyl benzoic acid.

It is noted that the steps of the preparation of a biochip are preferably performed according to the following sequence: coding is first performed and then surface chemical modification is performed so as to better protect the modified surface of the biochip, which facilitates the subsequent application of the biochip. For example, after the 2-dimensional bar code is printed on the chip using laser printing, the chip undergoes pretreatment and silanization treatment, and finally the surface of the chip is chemically modified; if the sequence is inverted, e.g. the surface of the chip is chemically modified after the chip undergoes pretreatment and silanization treatment, and finally the 2-dimensional bar code is printed on the chip using laser printing, it is easy to cause damage on the chemical molecule modified on the surface of the chip in the process of laser printing, affecting the subsequent application of the chip.

It is well understood that when the target compound is synthesized, the chemical entity (linker molecule) carried on the chip contacts a reaction reagent each time, the monomer in the reaction reagent is allowed to link to the terminal of the linker molecule to prolong the linker molecule. Contact and reaction are repeated, so as to continuously prolong the length of the synthesized compound until the target compound is obtained.

In some preferred embodiments, it is needed to conveniently cut/separate the synthesized product (target compound) from the chip after the synthesis reaction on the chip is completed.

For example, when the target compound is DNA, the target compound is cut from the chip through ammonolysis reaction. In some preferred embodiments, a reagent for ammonolysis reaction may be selected from ammonia water, ammonia gas, methylamine or any combination thereof. In some preferred embodiments, ammonolysis reaction is carried out at a temperature selected from room temperature~120°C, for example room temperature~60°C, 60-90°C and 90-120°C. In some preferred embodiments, ammonolysis reaction may be carried out for 0.5 h~48 h, for example 0.5~2 h, 2~5 h, 5~10 h, 10-18 h and 18~24 h. In some preferred embodiments, the target compound (DNA) is isolated and purified after ammonolysis reaction. For example, the target compound (DNA) is isolated and purified using MOP, PAGE, PAGE Plus, HPLC or any combination thereof.

For example, when the target compound is RNA, the target compound is cut from the chip through ammonolysis reaction. In some preferred embodiments, a reagent for ammonolysis reaction is selected from ammonia water, ammonia gas, methylamine or any combination thereof. In some preferred embodiments, ammonolysis reaction is carried out at a temperature selected from room temperature~120°C, for example room temperature~60°C, 60-90°C and 90-120°C. In some preferred embodiments, ammonolysis reaction is carried out for 0.5 h~48 h, for example 0.5-2h, 2~5 h, 5~10 h, 10-18 h and 18~24 h. In some preferred embodiments, the target compound (RNA) is isolated and purified after ammonolysis reaction. For example, the target compound (RNA) is isolated and purified using PAGE, PAGE Plus, HPLC or any combination thereof. In some preferred embodiments, separation and purification are carried out in a RNase-free environment. For example, in some preferred embodiments, HPLC is used for purification, and none of the reagents and equipment which are used for separation and purification contains RNase (namely, RNase-free) to avoid RNase contamination.

For example, when the target compound is a polypeptide, the target compound may be cut from the chip through addition of an eluting agent. In some preferred embodiments, reagents (eluting agents) for elution may be selected from hydrofluoric acid, trifluoroacetic acid, tetrafluoroboric acid or any combination thereof. In some preferred embodiments, the elution reaction may be carried out at a temperature selected from room temperature~120°C, for example, room temperature~60°C, 60-90°C and 90-120°C. In some preferred embodiments, elution reaction may be carried out for 0.5 h~48 h, for example 0.5~2 h, 2~5 h, 5~10 h, 10-18 h and 18~24 h. In some preferred embodiments, the target compound (polypeptide) is isolated and purified after elution reaction. For example, the target compound (polypeptide) is isolated and purified via high performance liquid chromatography, affinity chromatography, capillary electrophoresis or any combination thereof.

Optionally, after the modification step, the chemical modification may be detected or quantitatively analyzed using a plurality of technical means in order to characterize the modification quality of the chip. For example, firstly, the DMT-protected hydroxyl on the terminal of the linker molecule is de-protected through adding an acid, the color of the collected solution is qualitatively observed, and red solution indicates linker connection is successful, namely, the chip is successfully modified. Further, the collected red solution is collected and quantified, namely, DMT quantification is performed through an UV-Visible spectrophotometer, the number of Linker molecules on the surface of the modified chip is determined to determine the loading capacity of molecules on the surface of the chip. Finally, the chip without DMT is coupled with the fluorescent monomer of 6-FAM, the coupled chip is cleaned and then placed under a fluorescent microscope for observing fluorescence, the quality of the modified chip may be determined by the presence, intensity and homogeneity of the fluorescence, namely, high fluorescence intensity of the chip surface and meanwhile homogenous fluorescence signal indicate good quality of the modified chip.

Biochip for synthesis of nucleic acids

The chip of the application may be used for synthesis of nucleic acids (DNA/RNA). General methods and principles for solid-phase synthesis of nucleic acids are known by those skilled in the art. In general, solid-phase synthesis of nucleic acids adopts a solid-phase phosphoramidite triester method by which a plurality of nucleotides are linked to a chip one by one in turn through a plurality of rounds of circular reactions to obtain a target product containing a plurality of nucleotides, wherein each round of circular reaction involves four steps: deprotection, activation/coupling, capping and oxidization.

In a specific embodiment of DNA/RNA synthesis, firstly, according to variety of monomers required for synthesis and requirement of synthesis throughput, chip coding modes are selected, and recognition and sorting schemes are firstly formulated, so as to fabricate biochips for DNA/RNA synthesis with different throughputs and corresponding recognizers, sorters and drive devices for controlling chip movement and reagent liquid introduction and discharging. The specific signals on the chip are recognized through the recognizer, the chips are sorted into a "reaction tank" containing a corresponding coupling monomer, then the driver is controlled to realize the following: a deprotection reagent is introduced into the reaction tank for deprotection, and the deprotection reagent is discharged after the reaction is completed; then a coupling reagent is introduced for coupling according to the to-be-coupled monomer corresponding to the reaction tank, and then the coupling reagent is discharged; finally a capping reagent and an oxidization reagent are added in sequence for capping and oxidization respectively, at this moment, one cycle is completed. Through procedure setting, this cycle is repeated continuously until the synthesis is completed, the chips are collected together to obtain a DNA/RNA library, or an individual DNA/RNA fragment is obtained by utilizing the recognition sorting technology (a flowchart is seen in Fig. 2).

In a specific embodiment of polypeptide synthesis, firstly, according to variety of amino acid monomers required for polypeptide synthesis and requirement of synthesis throughput, chip coding modes are selected, and recognition and sorting schemes are formulated, so as to fabricate biochips for polypeptide synthesis with different throughputs and corresponding recognizers, sorters and drive devices for controlling chip movement and reagent liquid introduction and discharging. The specific signals on the chip are recognized through the recognizer, the chips are sorted into a "reaction tank" containing a corresponding coupling amino acid monomer, then the driver is controlled to realize the following: a deprotection reagent is introduced into the reaction tank for deprotection, the deprotection reagent is discharged after the reaction is completed; then a coupling reagent is introduced for coupling according to the to-be-coupled amino acid monomer corresponding to the reaction tank, and then the coupling reagent is discharged; at this moment, one cycle is completed. Through procedure setting, this cycle is repeated continuously until the synthesis is completed; the chips are collected together to obtain a polypeptide library, or an individual polypeptide fragment is obtained by utilizing the recognition and sorting technology (a flowchart is seen in Fig. 3).

Biochip for biological detection

The biochip of the application may be used for biological detection, such as antigen- and antibody screening and disease diagnosis, especially for simultaneously detecting multiple biological samples. The antigen and antibody have a specific bonding ability, namely, one antigen can specifically bind to a corresponding antibody in affinity; the specific screening of antigen-antibody may be carried out by utilizing this specific affinity. The conventional biological detection chip can only detect one biological sample simultaneously, namely, if antibody A is to be elected, a to-be-screened antibody library passes through a chip modified with specific antigen A, and after elution and desorbing, the target antibody A is obtained. Alternatively, a plurality of antigen sites are modified on a single chip to achieve the purpose of high throughput screening, however, this kind of modification is complicated and difficult to distinguish.

Screening of antibody

The biochip of the application can realize simple high-throughput antibody screening. The technical applicational solution will be described illustratively by taking a modified chip capable of screening 1000 antibodies:

1000 pieces of chips (the size of the chip is 2 mm*2 mm, even 1 mm*1 mm, 0.5 mm*0.5 mm or less) are mixed, each chip is modified with a specific antigen, and then a solution of an antibody library is added to and immersed the mixed chips. Because the antigen and the antibody have a specific binding effect, after a period of contact and reaction, the specific antibody binds to the antigen on the corresponding chip and then the antibody library is removed, and a washing reagent is added to wash the chips to ensure that the remaining unbound antibodies are removed up. Subsequently, according to the 2-dimensional bar codes marked on the chips, the 1000 pieces of chips are sorted into 1000 different reaction wells, and then a desorption reagent is added to each reaction well containing the chip to desorb and separate the antibody bound on the chip from the antigen on the chip; the above steps are repeated many times to ensure that the antibody is completely desorbed. Then the solutions in the 1000 reaction wells are transferred separately to new 1000 reaction wells, so that the corresponding 1000 specific antibodies will be screened at the same time, and finally the obtained antibodies are analyzed and characterized. The antibodies with positive results are purified to obtain high-purity antibodies. Through the above technical solution, a large number of high-purity specific antibodies are obtained rapidly and efficiently, so as to complete the high-throughput screening of the antibodies.

In the application of antibody screening proposed by the invention, since the chip subjected to antigen modification is tiny in volume and simple to fabricate, and may be produced on large scale; in conjunction with marking 2-dimensional bar codes on the chips, rapid and efficient recognition and sorting are performed, and the throughput of the technical application for antibody screening can be over ten thousand or even million levels.

Detection of antigen

The biochip of the application achieves simple and high-throughput antigen detection. The specific technical applicational solution will be described illustratively by taking the modified chips capable of detecting 1000 antigens as an example:

1000 pieces of chips (the size of the chip is 2 mm*2 mm, even 1 mm*1 mm, 0.5 mm*0.5 mm or less) are mixed, each chip is modified with a specific antibody, and then a solution of an antigen library is added to and immersed the mixed chips. Because the antigen and the antibody have a specific binding effect, after a period of contact and reaction, the specific antigen binds to the antibody on the corresponding chip and then the antigen library is removed, and a washing reagent is added to wash the chips to ensure that the remaining unbound antibodies are removed up. Subsequently, according to the 2-dimensional bar codes marked on the chips, the 1000 pieces of chips are sorted into 1000 different reaction wells, and then a desorption reagent is added to each reaction well containing the chip to desorb and separate the antigen bound on the chip from the antibody on the chip; the above steps are repeated many times to ensure that the antibody is completely desorbed. Then the solutions in the 1000 reaction wells are transferred separately to new 1000 reaction wells, so that the corresponding 1000 specific antigens will be screened at the same time, and finally the obtained antigens are analyzed and characterized. Through the above technical solution, the high-throughput detection of the antigens is completed.

In the technical application proposed by the invention, since the chip is tiny in volume and simple to fabricate, and may be produced on large scale; in conjunction with marking 2-dimensional bar codes on the chips, rapid and efficient recognition and sorting are performed, and the throughput of the technical application for antigen detection can be over ten thousand or even million levels.

The application will be described in detail though the following examples, wherein the main reagents and consumable materials used in the examples are as follows:

Solid phase carrier for synthesis: conventional chip, 100 nm silicon oxide wafer, 300 nm silicon oxide wafer, frosted quartz chip, transparent quartz chip, size: 2*2*0.45 mm
ACN (acetonitrile): Beijing Dina Xingke
Deprotection reagent: 3% TCA Deblock, Beijing Dina Xingke
Activator: 0.25 M Activator, Beijing Dina Xingke
Phosphoramidite monomers A, T, C, G: Sigma Aldrich
Oxidizing agent: 0.05 M Oxidizing, Beijing Dinaxingke
CAP A: Acetic anhydride/pyridine/tetrahydrofuran 1/1/8, Beijing Dinaxingke
CAP B: 17.6% w/v nitrogen-methyl imidazole/acetonitrile, Beijing Dina Xingke
Ammonia water: Sinopharm
T30 standard sample: Shenzhen National Gene Bank
TA cloning kit: pMDTM19-T, TaKaRa

Example 1 Preparation of a chip for synthesis of nucleic acids (DNA/RNA)

First of all, Guangruite Electronics was entrusted to perform printing and cutting of customized 2-dimensional bar code chip. Red laser was used for printing, the size of 2-dimensional bar code was 1 mm * 1 mm, a naked silicon wafer printed with 2-dimensional bar codes was cut into 2 mm * 2 mm * 0.45 mm microchips, the center of each microchip was printed with specific 2-dimensional bar codes, and the chips which had been printed with 2-dimensional bar code and cut were pretreated with piranha solution, and then cleaned with plasma. After that, the pretreated chips were silanized through simple negative pressure vapor deposition by using APTMS. Finally, the Universal linker purchased from Wuhu Huaren technology was coupled to the chips.

After that, the DMT protected hydroxyl at the terminal of the linker molecule was deprotected by adding acid, and the color of the collected solution was qualitatively observed. If the solution was red, it indicated that the Linker was successfully linked, that is, chip modification was successful. Then, the collected red solution was quantified, that is, DMT was quantified by UV-Visible spectrophotometer to determine the molecular number of the Linker on the surface of the modified chip, so as to determine the molecular load capacity on the surface of the chip. Alternatively, the chip without DMT was coupled with the fluorescent monomer of 6-FAM, the coupled chip was cleaned and then placed under the fluorescent microscope for observing fluorescence, the quality of the modified chip may be further qualitatively by the presence, intensity and homogeneity of the fluorescence, that is, high fluorescence intensity of the chip surface and homogenous fluorescence signal indicate good quality of the modified chip.

Specific modification steps are as follows:

1. 100 mL of 0.1 M NaOH solution was prepared and put into two 100 mL beakers. 2000 pieces of 2-dimensional bar code conventional chips were put into each beaker. The chips were washed for 3 min by shaking the breakers by hand. Firstly, the chips were washed with deionized water three times and acetone three times, and dried in the air, to make the surface of the chip clean to facilitate subsequent silanization modification.
2. 100 mL of 50% silanizing reagent was prepared and put into two 100 mL beakers. 2000 pieces of cleaned 2-dimensional bar code chips were put into each beaker, subjected to ultrasonic treatment in an ultrasonic instrument for 30 min, washed with acetone for 5 times and dried in the air;
wherein, 50% silanizing reagent was 1% (APTES: PTES = 1:1) acetone solution; taking the total volume of 100 mL as an example, the components were 500 μL of APTES, 500 μL of PTES and 99 mL of acetone.
3. The chips were dried at 80 °C for 10 min in a drying oven and taken out for subsequent experiments.
4. 1000 mg of Linker, 800 mg of HATU and 2000 μL of DIPEA were taken and put into 100 mL of acetonitrile, shaken uniformly and then put into two 50 mL centrifuge tubes. 2000 pieces of silanized 2-dimensional bar code chips were put into each centrifuge tube, and stirred in a vertical mixer overnight. After the reaction was completed, the chips were collected, washed with acetonitrile and acetone three times respectively, and dried in the air.
After that, 30 pieces of modified 2-dimensional bar code chips were taken out, and the DMT on the surface of the chip was eluted by TCA deblock. The concentration of DMT was measured by UV-vis spectrophotometer, and the grafting density of the Linker on the modified 2-dimensional bar code chip was determined.
5. The modified and dried chips were collected into a 50 mL centrifuge tube for later use.

Meanwhile, the effects of 1% and 100% silanizing reagents on chip modification were compared. The steps were the same as above, except that 1% and 100% silanizing reagents were used respectively to replace 50% silanizing reagents. 1% silanizing reagent was 1% (APTES: PTES = 1:99) acetone solution; taking the total volume of 100 mL as an example, the components were 10 μL of APTES, 990 μL of PTES and 99 mL of acetone. 100% silanizing reagent was 1% (APTES: PTES = 1:0) acetone solution; taking the total volume of 100 mL as an example, the components were 1000 μL of APTES, 0 μL of PTES and 99 mL of acetone. Finally, the grafting densities of Linker on the chips modified with three different silanization ratios were obtained. Results are shown in Table 1.

Considering that the grafting density of Linker on the surface of the chip is not positively related to the actual synthesis effect of the target compound, that is, high grafting density will not necessarily bring forth good synthesis effect of the chip because it involves the problem of molecular crowding. It is required to ensure the enough number of molecules on the surface of the chip while considering that the linked oligodeoxynucleotide chains are not too crowed to affect the final synthesis quality and length in the practical use. Therefore, the grafting density of Linker, single-step synthesis efficiency, product purity and other factors need to be comprehensively considered when exploring the optimal ratio of silanizing reagents in this example. In this example, after obtaining the chips modified with different silanizing reagent ratios, the chips were used to synthesize the T5 DNA sequence (namely, TTTTT (SEQ ID NO.15)). The specific method is seen in Example 3, and the purity of the final product and single-step synthesis efficiency are seen in Table 1. It can be seen from

the results in Table 1 that when 50% silanizing reagent was used for modification, although the grafting density of Linker was not as high as that of 100% silanizing reagent, the purity of the final product and single step synthesis efficiency of the obtained product were the highest.

Table 1

| Silane (APTES/PTES) ratio | Pieces | Linker grafting density (pmol/piece) | Synthetic sequence | HPLC purity (%) | Single-step synthesis efficiency (%) |
|---|---|---|---|---|---|
| 1% | 30 | 19.3 | T5 | 79.7 | 95.6 |
| 50% | 30 | 32.0 | T5 | 95.4 | 99.1 |
| 100% | 30 | 33.5 | T5 | 94.5 | 98.9 |

Example 2 Preparation of a chip for synthesis of polypeptides

First of all, Guangruite Electronics was entrusted to perform printing and cutting of a customized 2-dimensional bar code chip. Red laser was used for printing, the size of 2-dimensional bar code was 1 mm * 1 mm, a naked silicon wafer printed with 2-dimensional bar codes was cut into 2 mm * 2 mm * 0.45 mm microchips, the center of each microchip was printed with a specific 2-dimensional bar code, and the chips which had been printed with 2-dimensional bar codes and cut were pretreated with piranha solution, and then cleaned with plasma. After that, the pretreated chips were silanized through simple negative pressure vapor deposition by using APTMS. Finally, 4-hydroxymethyl benzoic acid purchased from Annaiji was coupled to the chip as the Universal linker.

Example 3 Synthesis of DNA

In this example, the inventors used the DNA synthesis method as shown in Fig. 2 and four single base reaction tanks to synthesis DNA molecules. The specific steps are as follows:

S1. Preparation of synthetic monomers, reaction reagents and reaction tanks
Four reaction tanks (first, second, third and fourth reaction tanks) were provided for adding deoxyribonucleotide A, T, C and G respectively, and for deprotection, capping, oxidation and washing in each reaction tank; wherein,

- when coupling,

adenylate with 5'-hydroxyl protected by DMT and 3'-hydroxyl protected by phosphoramidite group, and tetrazole were added in the first reaction tank;
guanosine acid with 5'-hydroxyl protected by DMT and 3'-hydroxyl protected by phosphoramidite group, and tetrazole were added in the second reaction tank;
cytidine acid with 5'-hydroxyl protected by DMT and 3'-hydroxyl protected by phosphoramidite group, and tetrazole were added in the third reaction tank;
thymidine acid with 5'-hydroxyl protected by DMT and 3'-hydroxyl protected by phosphoramidite group, and tetrazole were added in the fourth reaction tank;

- when in deprotection, dichloromethane solution of trichloroacetic acid was added into the four reaction tanks as a deprotection reagent;
- when in capping, acetonitrile solution of acetic anhydride/pyridine/tetrahydrofuran and N-methyl imidazole was added into the four reaction tanks as a capping reagent;
- when in oxidation, 0.01 M iodine solution was added into the four reaction tanks as an oxidant;
- when in washing, acetonitrile was added into the four reaction tanks as a cleaning solution.

S2. Preparation of chips
Five different types of chips (conventional chip, 100 nm silicon oxide chip, 300 nm silicon oxide chip, frosted quartz chip and transparent quartz chip) modified with 50% silanizing reagent in Example 1 were used.
S3. Preparation of equipment and software programs
Equipment was provided, wherein the equipment included,

- 2-dimensional bar code recognizer, which recognized the 2-dimensional bar code carried on the chip and

generated a signal;

- chip sorter, which sorted the chips;
- driving device, which drove the movement of chips;
- reagent liquid driver, which introduced and discharged the reagent in various "tanks";
- central processing unit, which received a signal generated by the 2-dimensional bar code recognizer after a 2-dimensional bar code was recognized, and sent an instruction to control the chip sorter to sort chips and/or control the driving device to move the chip; and
- memory, which was used to store the sequences of DNA molecules to be synthesized, corresponding relationships between the chips and 2-dimensional bar codes, corresponding relationships between the sequences of DNA molecules to be synthesized and the chips, and the reactions experienced by each solid-phase matrix.

In addition, a control program was designed according to the sequence of the target DNA to be synthesized, which determined the chip sorting scheme according to the predetermined DNA sequence.

S4. DNA synthesis

Firstly, all chips were mixed, and the 2-dimensional bar codes printed on the chips in advance were recognized by the recognizer. The central processing unit controlled the chip sorting device to sort the chips according to the recognition signals generated by the recognizer, controlled the driving device to move each chip to the desired reaction tank according to the preset control program, and then controlled the reagent liquid driver to realize the following: the deprotection reagent was introduced into the reaction tank for deprotection, the deprotection reagent was discharged after the reaction was completed, the cleaning solution was introduced to clean the chips, and then the coupling reagent was introduced for coupling according to the monomer corresponding to the reaction tank, the coupling reagent was discharged after the reaction was completed, the cleaning solution was introduced to clean the chips, and finally, the capping reagent, the oxidizing reagent and the cleaning solution were introduced again in turn for capping, oxidization and cleaning respectively; at this moment, one cycle was completed, i.e. addition/coupling of one base was completed. Specific operation steps, reagents used and reaction time are shown in Table 2.

After the coupling of one base was completed, the above steps were repeated according to the predetermined DNA synthesis sequence information until DNA molecule synthesis was completed.

Table 2

| Operation steps | Reagent/solvent | Reagent amount | Immersion time | Reaction times |
|---|---|---|---|---|
| Deprotection | 3% trichloroacetic acid /dichloromethane | 150 μL | 15 s | 2 |
| Washing | Acetonitrile | 250 μL | 10 s | 3 |
| Coupling | 0.1 M phosphoramidite monomer/acetonitrile, 0.5 M tetrazole/acetonitrile | 40 μL (T)+60 μL (ACT) | 60 s | 2 |
| Washing | Acetonitrile | 250 μL | 10 s | 1 |
| Capping | Acetic anhydride/pyridine/ tetrahydrofuran 1/1/8, 17.6% w/v nitrogen-methyl imidazole /acetonitrile | 75 μL (A)+75 μL (B) | 20 s | 2 |
| Washing | Acetonitrile | 250 μL | 10 s | 1 |
| Oxidizing | 0.01 M iodine solution (water/pyridine/tetrahydrofur an 2/20/78) | 150 μL | 20 s | 2 |
| Washing | Acetonitrile | 250 μL | 10 s | 3 |

S5. Separation and purification of DNA

After DNA synthesis was completed, all the chips were collected together for ammonolysis to separate and purify all the synthesized DNA molecules. Alternatively, all the chips can be recognized using the 2-dimensional bar codes, the target chips were sorted and collected and subjected to ammonolysis, thereby separating and purifying the target DNA molecules.

In this example, five different types of chips were selected for T30 DNA (sequence: TTTTTTTTTT TTTTTTT TTTTTTTTTTTTT (SEQ ID No.16)) synthesis, in which the five chips were: conventional chip, 100 nm oxidation chip, 300

nm oxidation chip, frosted quartz chip and transparent quartz chip. The specific steps are as follows:

(1) Experiment 1-T30

40 pieces of conventional chips (specification: 2*2*0.45 mm, both of two sides having 2-dimensional bar codes) were taken and mixed, and the monomer to be added in this cycle was determined according to the target sequence of synthesis. After sorting was performed with the chip sorter, the chips for synthesis with the same monomer were sorted into the same reaction tank, the capping reagent consisting of 75 μL of Cap A and 75 μL of Cap B was added. The chips were immersed in the reaction tank once, the capping reagent was discharged, and the chips were soaked for reaction once again after adding unused capping reagent once again, and the capping reactions were performed for 40 s in total; then the capping reagent was discharged, and acetonitrile was added for washing 3 times. Used acetonitrile was discharged and 150 μL of deprotection reagent TCA was added, and deprotection reaction was performed for 15s. After the used deprotection reagent was discharged, unused deprotection reagent was added again to react once for 15 s. After the deprotection step was completed, the used deprotection reagent was discharged and 250 μL of acetonitrile was added for washing 3 times. Acetonitrile was discharged and then 40 μL of phosphoramidite monomer T and 60 μL of activator ACT were added, and coupling reaction was carried out for 60 s. After the used reagent was discharged, unused reagent was added once again to react, and the two reactions were performed for 120 s in total. The coupling step was completed. The used coupling reagent was discharged, 250 μL of acetonitrile was added for washing once. Acetonitrile was discharged and the capping reagent consisting of 75 μL of cap A and 75 μL of cap B was added. The capping reaction was performed once. After the used capping reagent was discharged, unused capping reagent was added again once, and the two capping reactions were performed for 40 s in total. The used capping reagent was discharged and 250 μL of acetonitrile was added for washing once. The acetonitrile was discharged and 150 μL of oxidant was added, and the oxidation reaction was performed for 20 s. After the used reagent was discharged, unused reagent was added again once. The two oxidation reactions were performed for 40 s in total. The used oxidation reagent was discharged and 250 μL of acetonitrile was added for three times. One cycle was completed at this moment. The above steps were recycled for 30 times, and then finally deprotection was performed, ammonolysis was performed with ammonia water, and the T30 product was obtained after treatment. The single-chip synthesis quantity was quantitatively measured by Nanodrop, and the HPLC purity was measured by HPLC analysis. The single-step synthesis efficiency was calculated (the single-step synthesis efficiency was obtained by HPLC purity analysis. For example, if the HPLC purity of the synthetic T10 was 80%, the single-step synthesis efficiency was $\sqrt[10]{0.8} = 97.8\%$). The HPLC results are seen in Fig. 4A.

Furthermore, it should be noted that before the first cycle of deprotection, capping treatment was carried out to seal the area on the surface of the chip where the end groups were not completed, so that it couldn't couple the building units, thereby improving the synthesis efficiency. Starting from the second cycle, there is no need for capping again, but the "deprotection-coupling-capping-oxidation" steps were performed from recognition and sorting. The cycle was performed until the completion of synthesis.

(2) Experiment 2-T30

40 pieces of 100 nm oxidized chips were taken to synthesize T30 DNA. The specific steps were the same as Experiment 1. The results of HPLC are seen in Fig. 4B.

(3) Experiment 3-T30

40 pieces of 300 nm oxidation chips were taken to synthesize T30 DNA. The specific steps were the same as Experiment 1. The results of HPLC are seen in Fig. 4C.

(4) Experiment 4-T30

40 pieces of frosted quartz chips were taken to synthesize T30 DNA. The specific steps were the same as Experiment 1. The results of HPLC are seen in Fig. 4D.

(5) Experiment 5-T30

40 pieces of transparent quartz chips were taken to synthesize T30 DNA. The specific steps were the same as Experiment 1. The results of HPLC are seen in Fig. 4E.

The DNA synthesis results of the above five experiments are summarized, and the results are shown in Table 3. It

can be seen from Table 3 that for DNA synthesis performed using the transparent quartz chip, the purity of the product and single-step synthesis efficiency were the highest. Relative to 100 nm oxidized chip with the lowest single-step synthesis efficiency, the single-step synthesis efficiency was increased by 2.5%, that is, the synthesis efficiency of each cycle was increased from 96.8% to 99.3%, and the whole synthesis process needed 30 cycles, so the purity of the product was significantly improved from 37.9% to 81.0% after the whole synthesis process. When the synthesized sequence is longer and the number of reaction cycles is more, the purity of the product is improved more significantly.

Table 3

| Experiment number | Chips | Object picture | Amount of single piece synthesis | HPLC purity | Single-step synthesis efficiency |
|---|---|---|---|---|---|
| 1 | Conventional chip | 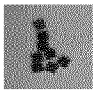 | 15.7 pmol | 43.0% | 97.2% |
| 2 | 100 nm oxidized chip | 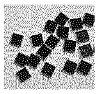 | 9.2 pmol | 37.9% | 96.8% |
| 3 | 300 nm oxidized chip | 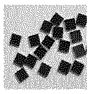 | 10.5 pmol | 55.4% | 98.1% |
| 4 | Frosted quartz chip | 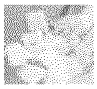 | 54.6 pmol | 71.3% | 98.9% |
| 5 | Transparent quartz chip | 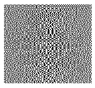 | 26.8 pmol | 81.0% | 99.3% |

(6) Experiment 6: 59 nt oligonucleotide synthesis based on transparent quartz chip, gene assembling test, and sequencing results analysis

In order to further identify the optimal carrier for synthesis, the transparent quartz chip was used to synthesize 14 pieces of 59 nt oligonucleotides, and the sequences are shown in Table 4. The correctness of the target band was verified by small fragment gene assembly based on one-step PCA/PCR reaction strategy and gel electrophoresis image. Finally, the optimal solid-phase synthesis carrier was identified after gel cutting and recovery, TA cloning transformation and Sanger sequencing.

Table 4

| Number | Sequence | SEQ ID NO |
|---|---|---|
| 59 nt-1 | GCCCATTTCTCGAACGTACTTCTCACAGTTTCAGTTAAGAAATCGAAGAAGTAAGATAC | 1 |
| 59 nt-2 | AGTACGTTCGAGAAATGGGCCACTACCGGAGAAACACTTCAGTTACTCTTTGAGATGAA | 2 |
| 59 nt-3 | AAGTATTTGCATAAGCACTTTAAATGTAGGTTTTTTCATCTCAAAGAGTAACTGAAGTG | 3 |
| 59 nt-4 | ACATTTAAAGTGCTTATGCAAATACTTATAGGTGGCAATCAAGTTGAAAATGAATTGCT | 4 |
| 59 nt-5 | CAAATCGGAAATTGTTCTCCTTGAACAAAGCGTCAAGCAATTCATTTTCAACTTGATTG | 5 |
| 59 nt-6 | CAAGGAGAACAATTTCCGATTTGCTGGTCTTCGTAGCATGCCCCTCGATTTCCCCGGAT | 6 |
| 59 nt-7 | AACTATTTCTCCTCGACCCTTCATTGCCCTGTTGTAAGTAGATCCGGGGAAATCGAGGG | 7 |
| 59 nt-8 | GAAGGGTCGAGGAGAAATAGTTAAGATATACGAACGGATAATAAATGAAAGAAAAGTGA | 8 |
| 59 nt-9 | GGTCCTTGGCTCTCCTCTAGTCTTGGCAATCATCACTTTTCTTTCATTTATTATCCGTT | 9 |
| 59 nt-10 | TAGAGGAGAGCCAAGGACCAACATACTGGATATAATGCTAGACAGTCAATGTGATGAGG | 10 |

(continued)

| Number | Sequence | SEQ ID NO |
|---|---|---|
| 59 nt-11 | ACTTTCATGATATTCTCATCATTCAATACTTTTCCTTCCTCATCACATTGACTGTCTAG | 11 |
| 59 nt-12 | GTATTGAATGATGAGAATATCATGAAAGTGCTGCTTTGGTATACATTTAGTGGTTATGA | 12 |
| 59 nt-13 | ATAATAGTCTGTGTGGCAACCTTTGCAATAGATTCATAACCACTAAATGTATACCAAAG | 13 |
| 59 nt-14 | AGGTTGCCACACAGACTATTATGTTATTGGAAAAACACCCCGAGTGCTTCCAGAAAGCA | 14 |

The specific steps are as follows:
42 pieces of transparent quartz chips were taken to synthesize the oligonucleotides according to the method of Experiment 1 in Example 3. Deprotection was performed to synthesize 14 oligonucleotides of which the sequences are listed in the above table. Each oligonucleotide was synthesized in parallel by using 3 pieces of chips (that is, each sequence was synthesized by using 3 groups of parallel tests), and 42 oligonucleotides were synthesized in total. 42 primers 3 * (59 nt-1 - 59 nt-14) were divided into three groups (14 pieces/group) for ammonolysis. After treatment, three groups of 59 nt mix products in 50 μL each were obtained. 10 μL of samples were added with 4 μL of dNTPs, 5 μL of buffer, 4 μL of head tail primers and 0.5 μL of DNA polymerase respectively. The volume was supplemented to 50 μL with water, and the mixture was evenly mixed for one-step PCA/PCR reaction. After 35 cycles of PCR amplification, the products were stored at 12 °C. 2 μL of PCR products were spotted into the gel well, the voltage was adjusted to 180 V, and the electrophoresis time was 30 min. The results are shown in Fig. 5. It can be seen from Fig. 5 that the 560 bp gene band assembled by the 59 nt primers synthesized on the transparent quartz chips was clear and correct compared with the standard synthetic primers.

In addition, the gel with PCR products with correct bands were cut and recovered, and then a TA cloning Kit (PMDTM19-T) of TaKaRa Company was used for cloning and transformation experiment. TA clone transformants with correct bands that had been verified by Colony PCR were sent to Sanger sequencing. Results of Sanger sequencing are shown in Table 5. The average error rate of the three groups of parallel experiments was 0.34%.

Table 5

| Types of chip | Primer | Deletion | Mutation | Insertion | Total error number | Gene length | Total base number | Error rate |
|---|---|---|---|---|---|---|---|---|
| Transparent quartz chip | Mix-1 | 5 | 2 | 6 | 13 | 560 | 3920 | 0.33% |
| | Mix-2 | 6 | 2 | 6 | 14 | 560 | 4480 | 0.31% |
| | Mix-3 | 6 | 6 | 5 | 17 | 560 | 4480 | 0.38% |

By chemical modification to different types of 2-dimensional bar code chips and a series of 59 nt oligonucleotide synthesis based on soaking-recognizing-sorting on the modified chips and gene synthesis and sequencing, and by comparing the HPLC analysis results of synthesis of a plurality of chips , it is indicated that the 2-dimensional bar code chips modified by chemical modification can be used to synthesize oligonucleotides, and the transparent quartz chip has the best synthesis effect. The single-chip synthesis amount of corresponding T30 primers was 26.8 pmol, the HPLC purity was 81.0%, and the single-step synthesis efficiency reached 99.3%. Finally, synthesis of 14 of 59 nt primers, small fragments assembly and Sanger sequencing further verified that the transparent quartz chip had better effect, and the error rate of the sequencing results of Mix group was lower to 0.3%.

The above examples and experimental results show that the biochip proposed by the invention can be used for DNA synthesis, and the final test results show that the transparent quartz chip has good synthesis effect, which is comparable with the current commercial mainstream synthesis. It is further showed that the chemical modified biochip proposed by the invention is feasible and has a good application prospect.

Example 4 Synthesis of RNA

In this example, the inventors used an RNA synthesis method based on soaking-sorting and 4 single-base reaction tanks for synthesis of RNA molecules. The specific steps are as follows:

S1. Preparation of synthetic monomers, reaction reagents and "tanks"
Four tanks (first, second, third and fourth reaction tanks) were provided for adding ribonucleotides A, U, C and G respectively, and for deprotection, capping, oxidation and washing in each reaction tank; wherein,

- when coupling,

  adenylate with 5'-hydroxyl protected by DMT, 2'-hydroxyl protected by TBDMS and 3'-hydroxyl protected by phosphoramidite group, and tetrazole were added in the first reaction tank;
  guanosine acid with 5'-hydroxyl protected by DMT, 2'-hydroxyl protected by TBDMS and 3'-hydroxyl protected by phosphoramidite group, and tetrazole were added in the second reaction tank;
  cytidine acid with 5'-hydroxyl protected by DMT, 2'-hydroxyl protected by TBDMS and 3'-hydroxyl protected by phosphoramidite group, and tetrazole were added in the third reaction tank;
  thymidine acid with 5'-hydroxyl protected by DMT, 2'-hydroxyl protected by TBDMS and 3'-hydroxyl protected by phosphoramidite group, and tetrazole were added in the fourth reaction tank;

- when in deprotection, dichloromethane solution of trichloroacetic acid was added into the four reaction tanks as a deprotection reagent;
- when in capping, acetonitrile solution of acetic anhydride/pyridine/tetrahydrofuran and N-methyl imidazole was added into the four reaction tanks as a capping reagent;
- when in oxidation, 0.01 M iodine solution was added into the four reaction tanks as an oxidant;
- when in washing, acetonitrile was added into the four reaction tanks as a cleaning solution.

S2. Preparation of chips
Chips modified by 50% silanizing reagent in Example 1 were used.
S3. Preparation of equipment and software programs
Equipment was provided, wherein the equipment included,

- 2-dimensional bar code recognizer, which recognized the 2-dimensional bar code carried on the chip and generated signals;
- chip sorter, which sorted the chips;
- driving device, which drove the movement of chips;
- reagent liquid driver, which introduced and discharged the reagents in various "tanks";
- central processing unit, which received a signal generated by the 2-dimensional bar code recognizer after a 2-dimensional bar code was recognized, and sent an instruction to control the chip sorter to sort chips and/or control the driver to move the chip; and
- memory, which was used to store the sequences of RNA molecules to be synthesized, corresponding relationships between the chips and 2-dimensional bar codes, corresponding relationships between the sequences of RNA molecules to be synthesized and the chips, and the reactions experienced by each chip.

In addition, a control program was designed according to the sequence of the target RNA to be synthesized, which determined the chip sorting scheme and moving scheme according to the predetermined RNA sequences.

S4. RNA synthesis

Firstly, 1000 pieces of chips were mixed, and the 2-dimensional bar codes printed on the chips in advance were recognized by the recognizer. The central processing unit controlled the chip sorting device to sort the chips according to the recognition signals generated by the recognizer, controlled the driving device to move each chip to the desired reaction tank according to the predetermined control program, and then controlled the reagent liquid driver to realize the following: the deprotection reagent was introduced into the reaction tank for deprotection, the deprotection reagent was discharged after the reaction was completed, the cleaning solution was introduced to clean the chips, and then the coupling reagent was introduced for coupling according to the monomer corresponding to the reaction tank, the coupling reagent was discharged after the reaction was completed, the cleaning solution was introduced to clean the chips, and finally, the capping reagent, the oxidizing reagent and the cleaning solution were introduced again in turn for capping, oxidization and cleaning respectively; at this moment, one cycle was completed, i.e. addition/coupling of one base was completed. The specific operation steps, reagents used and reaction time are shown in Table 6.
After one coupling of base was completed, the above steps were repeated according to the predetermined RNA synthesis sequence information until synthesis of 1000 RNA molecules was completed.

Table 6

| Operation steps | Reagents/solvents | Soaking time |
|---|---|---|
| Deprotection | 10% trichloroacetic acid/dichloromethane | 15 s |

(continued)

| Operation steps | Reagents/solvents | Soaking time |
|---|---|---|
| Cleaning | Acetonitrile | 60 s |
| Cleaning | Acetonitrile | 60 s |
| Coupling | 0.1 M phosphoramidite monomer (single base) /acetonitrile, 0.5 M tetrazole/acetonitrile | 90 s |
| Cleaning | Acetonitrile | 60 s |
| Capping | Acetic anhydride /pyridine/tetrahydrofuran 1/1/8, 17.6% w/v nitrogen-methyl imidazole/acetonitrile | 30 s |
| Oxidizing | 0.01 M iodine solution (water/pyridine/tetrahydrofuran 2/20/78) | 30 s |
| Cleaning | Acetonitrile | 60 s |
| Cleaning | Acetonitrile | 60 s |

S5, separation and purification of RNA

After RNA synthesis was finished, 1000 pieces of chips were collected together for ammonolysis to separate and purify all the synthesized RNA molecules. Alternatively, 1000 pieces of chips can be recognized using the 2-dimensional bar codes, the target chips were sorted and collected and subjected to ammonolysis, thereby separating and purifying the target RNA molecules.
The RNA molecules synthesized by each chip was separated, purified and sequenced. It was shown by the sequencing results that each synthesized RNA molecule had an expected target sequence.

Example 5 Synthesis of polypeptide

In this example, the inventors used a polypeptide synthesis method based on soaking-sorting as shown in Fig. 3 and 21 single amino acid reaction tanks for synthesis of polypeptides. The specific steps are as follows:

S1. Preparation of synthetic monomers, reaction reagents and "tanks"
23 tanks were provided, wherein,

- 21 synthesis tanks (corresponding to 21 conventional amino acid monomers), which were provided for adding a different amino acid monomer respectively, wherein each synthesis tank contained an amino acid monomer with an $\alpha$-amino group protected by Fmoc and a side chain protected (if a side chain was present), as well as tetramethyluronium hexafluorophosphate, N-methyl morpholine and DMF;
- a deprotection tank, in which DMF solution containing 20% piperidine was used as a deprotecting agent;
- a washing tank, which contained DMF/DCM as a cleaning solution.

S2. Preparation of chips
Chips prepared in Example 2 were used.
S3. Preparation of equipment and software programs
Equipment was provided, wherein the equipment included,

- 2-dimensional bar code recognizer, which recognized the 2-dimensional bar code carried on the chip and generated signals;
- chip sorter, which sorted the chips;
- driving device, which drove the movement of chips;
- reagent liquid driver, which introduced and discharged the reagents in various "tanks";
- central processing unit, which received a signal generated by the 2-dimensional bar code recognizer after a 2-dimensional bar codes was recognized, and sent an instruction to control the chip sorter to sort chips and/or control the driving device to move the chip; and
- memory, which was used to store the sequences of peptides to be synthesized, corresponding relationships between the chips and 2-dimensional bar codes, corresponding relationships between the sequences of peptides to be synthesized and the chips, and the reactions experienced by each chip.

In addition, a control program was designed according to the sequence of the target peptide to be synthesized, which determined the chip sorting scheme and moving scheme according to the predetermined peptide sequence.

S4. Polypeptide synthesis

Firstly, 1000 pieces of chips were placed in the deprotection tank for deprotection; after the reaction was completed, they were moved to the washing tank for cleaning twice; and then the 2-dimensional bar codes printed on the chips in advance were recognized by the recognizer. The central processing unit controlled the chip sorting device to sort the chips according to the recognition signals generated by the recognizer, controlled the driving device to move each chip to the desired reaction tank for coupling reaction according to the predetermined control program. After the coupling reaction was completed, all of the chips were moved to the washing tank for cleaning twice, and thus addition/coupling of one amino acid was completed. In each reaction in the above steps, by using the reagent liquid driver, the corresponding reagent was introduced into the tank before starting the reaction and discharged out of the tank after the reaction was completed. The specific operation steps, reagents used and reaction time are shown in Table 7.

After the coupling of one amino acid was completed, the above steps were repeated according to the predetermined sequence information of the polypeptide until 1000 polypeptide molecules were synthesized.

Table 7

| Operation steps | Reagents/solvents | Soaking time |
|---|---|---|
| Deprotection | 20% piperidine/DMF | 15 s |
| Cleaning | DMF/DCM | 60 s |
| Cleaning | DMF/DCM | 60 s |
| Coupling | Amino acid monomer, tetramethyluronium hexafluorophosphate /N-methyl morpholine, DMF (1:24) | 90s |
| Cleaning | DMF/DCM | 60s |
| Cleaning | DMF/DCM | 60s |

S5. Separation and purification of polypeptides

After the polypeptide synthesis was finished, 1000 pieces of chips were collected together for dissociation to separate and purify all the synthesized polypeptide molecules. Alternatively, 1000 pieces of chips were recognized using the 2-dimensional bar codes, the target chips were sorted and collected and subjected to dissociation, thereby separating and purifying the target polypeptide molecules.

The polypeptide molecules synthesized by each chip were separated, purified and sequenced. It was shown by the sequencing results that each synthesized polypeptide molecule had an expected target sequence.

## Claims

1. A biochip carrying a chemical entity and a code, wherein the code has a unique corresponding relationship with the biochip; wherein the biochip has a surface treated with a silanizing reagent, and the silanizing reagent is a 50% silanizing reagent; and wherein the chemical entity is a linker molecule capable of initiating a DNA/RNA synthesis reaction, and the biochip is made of silicon wafer.

2. The biochip according to claim 1, wherein
the chemical entity is a linker molecule having a functional group reacting with an amino group on a beginning end and having hydroxyl protected with an acid-labile protecting group on a terminal, more preferably Universal Linker.

3. The biochip according to any one of claims 1~2, wherein the code is a specific characterization for the identity of the biochip, comprising a number, a symbol, a graph and/or an identification code; preferably, the code is a 2-dimensional bar code.

4. The biochip according to any one of claims 1~3, wherein
the biochip is selected from a non-polished single-sided 2-dimensional bar code chip, a non-polished double-sided 2-dimensional bar code chip, a single-polished single-sided 2-dimensional bar code chip, a single-polished double-sided 2-dimensional bar code chip, a double-polished single-sided 2-dimensional bar code chip and a double-polished double-sided 2-dimensional bar code chip.

**5.** The biochip according to any one of claims 1~3, wherein the biochip has a size of less than 2 mm*2mm, preferably 0.5 mm*0.5mm.

**6.** The biochip according to any one of claims 1~3, wherein the biochip is made of porous glass, and the porous glass has a particle size of 5 μm-2000 μm; the porous glass has a pore diameter of 200Á-5000Á.

**7.** The biochip according to any one of claims 1~3, wherein the biochip is selected from a 100 nm silicon oxide wafer, a 300 nm silicon oxide wafer, a frosted quartz chip or a transparent quartz chip.

**8.** The biochip according to any one of claims 1~3, wherein the biochip is a transparent quartz chip.

**9.** The biochip according to any one of claims 1~3, wherein the silanizing reagent is selected from APTMS or APTES.

**10.** A method for preparing the biochip according to any one of claims 1~9, the method comprising the following steps:

1) coding the biochip;
2) pretreating the biochip;
3) performing silanization treatment on the surface of the biochip; wherein the silanization treatment is performed on the surface of the biochip by soaking the biochip in a silanizing reagent and carrying out ultrasonic treatment, and the silanizing reagent is a 50% silanizing reagent;
4) chemically modifying the surface of the biochip; and
5) optionally, detecting or quantitatively analyzing the chemical modification;

preferably, the pretreating comprises acid treatment, alkali treatment, ultrasonic treatment, plasma cleaning, acetone washing or a combination thereof, wherein the acid is selected from sulfuric acid, hydrochloric acid, phosphoric acid and piranha liquid, the alkali is selected from sodium hydroxide and potassium hydroxide;
preferably, the pretreatment method is acid treatment followed by plasma cleaning; more preferably, the pretreatment method is sodium hydroxide treatment followed by plasma cleaning and acetone washing;
preferably, the chemical modification on the surface of the biochip is achieved by linking the chemical entity on the surface of the biochip.

**11.** The method according to claim 10, wherein a 2-dimensional bar code is printed on the biochip by means of laser printing, preferably, coding is carried out using red laser through a dot 2-dimensional bar code mode.

**12.** The method according to claim 10, wherein the silanizing reagent comprises APTES and PTES.

**13.** Use of the biochip according to any one of claims 1~9 or the biochip prepared by using the method according to any one of claims 10~13 in synthesis of a nucleic acid.

**14.** A method for synthesizing a nucleic acid, comprising the following steps:

(1) providing the biochip according to any one of claims 1~9 or the biochip prepared by using the method according to any one of claims 10-13, wherein the code carried on the biochip corresponds to a sequence of the nucleic acid to be synthesized;
(2) providing four reaction tanks which are respectively used for addition of deoxyribonucleotides A, T, C and G or ribonucleotides A, U, C and G, and for deprotection, capping, oxidation and washing in each of the reaction tanks;
(3) recognizing the code carried on the biochip, and determining the deoxyribonucleotide or ribonucleotide to be added according to the sequence corresponding to the code, and sorting the biochips with the same deoxyribonucleotide or ribonucleotide to be added into the same reaction tank;
(4) adding a deprotection reagent in the reaction tank, soaking the biochip in the reaction tank for deprotection, and discharging the deprotection reagent after the reaction is completed;
(5) adding a coupling reagent of corresponding deoxyribonucleotide or ribonucleotide into the reaction tank, soaking the biochip in the reaction tank for coupling reaction, and discharging the coupling reagent after the reaction is completed;

(6) adding a capping reagent in the reaction tank, soaking the biochip in the reaction tank for capping reaction, and discharging the capping reagent after the reaction is completed;
(7) adding an oxidizing reagent in the reaction tank, soaking the biochip in the reaction tank for oxidizing reaction, and discharging the oxidizing reagent after the reaction is completed, thereby completing addition of one nucleotide;
(8) according to the sequence of nucleic acid to be synthesized corresponding to the code, repeating steps (3)~(7) once or more times, thereby generating a nucleic acid having a predetermined sequence on the biochip;
optionally, the method further comprises the following steps:
(9) cutting the nucleic acid from the biochip, thereby obtaining the nucleic acid;

**15.** The method according to claim 14, wherein before the nucleic acid synthesis and after the biochip is sorted to the reaction tank, the capping reagent is added in the reaction tank, and the biochip is soaked in the reaction tank for capping reaction;
preferably, a plurality of chips are simultaneously used to perform synthesis reaction.

## Patentansprüche

**1.** Biochip, der eine chemische Einheit und einen Code trägt, wobei der Code eine eindeutige korrespondierende Beziehung zu dem Biochip aufweist; wobei der Biochip eine mit einem Silanisierungsreagenz behandelte Oberfläche aufweist und das Silanisierungsreagenz ein 50 %iges Silanisierungsreagenz ist; und wobei die chemische Einheit ein Linkermolekül ist, das in der Lage ist, eine DNA/RNA-Synthesereaktion zu initiieren, und der Biochip aus einem Siliziumwafer gefertigt ist.

**2.** Biochip nach Anspruch 1, wobei
die chemische Einheit ein Linkermolekül ist, das eine funktionelle Gruppe aufweist, die mit einer Aminogruppe an einem Anfangsende reagiert und mit einer säurelabilen Schutzgruppe an einem terminalen Ende hydroxylgeschützt ist, vorzugsweise ein Universal Linker.

**3.** Biochip nach einem der Ansprüche 1 bis 2, wobei der Code eine spezifische Kennzeichnung für die Identität des Biochips ist, umfassend eine Zahl, ein Symbol, eine Grafik und/oder einen Identifikationscode; vorzugsweise wobei der Code ein zweidimensionaler Strichcode ist.

**4.** Biochip nach einem der Ansprüche 1 bis 3, wobei
der Biochip ausgewählt ist aus einem nicht polierten einseitigen 2-dimensionalen Strichcode-Chip, einem nicht polierten doppelseitigen 2-dimensionalen Strichcode-Chip, einem einseitig polierten einseitigen 2-dimensionalen Strichcode-Chip, einem einseitig polierten doppelseitigen 2-dimensionalen Strichcode-Chip, einem doppelseitig polierten einseitigen 2-dimensionalen Strichcode-Chip und einem doppelseitig polierten doppelseitigen 2-dimensionalen Strichcode-Chip.

**5.** Biochip nach einem der Ansprüche 1 bis 3, wobei der Biochip eine Größe von weniger als 2 mm*2mm, vorzugsweise 0,5 mm*0,5mm aufweist.

**6.** Biochip nach einem der Ansprüche 1 bis 3, wobei der Biochip aus porösem Glas gefertigt ist und das poröse Glas eine Teilchengröße von 5 μm bis 2000 μm aufweist; das poröse Glas einen Porendurchmesser von 200 Ǻ bis 5000 Ǻ aufweist.

**7.** Biochip nach einem der Ansprüche 1 bis 3, wobei der Biochip ausgewählt ist aus einem 100 nm Siliziumoxid-Wafer, einem 300 nm Siliziumoxid-Wafer, einem mattierten Quarzchip oder einem transparenten Quarzchip.

**8.** Biochip nach einem der Ansprüche 1 bis 3, wobei der Biochip ein transparenter Quarzchip ist.

**9.** Biochip nach einem der Ansprüche 1 bis 3, wobei das Silanisierungsreagenz ausgewählt ist aus APTMS oder APTES.

**10.** Verfahren zum Herstellen des Biochips nach einem der Ansprüche 1 bis 9, das Verfahren umfassend die folgenden Schritte:

1) Kodieren des Biochips;

2) Vorbehandeln des Biochips;

3) Durchführen einer Silanisierungsbehandlung auf der Oberfläche des Biochips; wobei die Silanisierungsbehandlung auf der Oberfläche des Biochips durchgeführt wird, indem der Biochip in einem Silanisierungsreagenz getränkt und eine Ultraschallbehandlung durchgeführt wird, und das Silanisierungsreagenz ein 50 %iges Silanisierungsreagenz ist;

4) chemisches Modifizieren des Biochips; und

5) optional Erkennen oder quantitatives Analysieren der chemischen Modifikation;

wobei die Vorbehandlung vorzugsweise eine Säurebehandlung, eine Alkalibehandlung, eine Ultraschallbehandlung, eine Plasmareinigung, eine Acetonwäsche oder eine Kombination davon umfasst, wobei die Säure ausgewählt ist aus Schwefelsäure, Salzsäure, Phosphorsäure und Piranha-Flüssigkeit und die Lauge aus Natriumhydroxid und Kaliumhydroxid; vorzugsweise das Vorbehandlungsverfahren eine Säurebehandlung mit anschließender Plasmareinigung ist; bevorzugter das Vorbehandlungsverfahren eine Natriumhydroxidbehandlung mit anschließender Plasmaaufreinigung und Acetonwäsche ist;

vorzugsweise die chemische Modifikation auf der Oberfläche des Biochips durch die Verknüpfen der chemischen Einheit auf der Oberfläche des Biochips erreicht wird.

**11.** Verfahren nach Anspruch 10, wobei ein zweidimensionaler Strichcode mittels Laserdruck auf den Biochip gedruckt wird, wobei das Kodieren vorzugsweise mit einem roten Laser durch einen punktförmigen zweidimensionalen Strichcodemodus durchgeführt wird.

**12.** Verfahren nach Anspruch 10, wobei das Silanisierungsreagenz APTES und PTES umfasst.

**13.** Verwendung des Biochips nach einem der Ansprüche 1 bis 9 oder des Biochips, der durch Verwenden des Verfahrens nach einem der Ansprüche 10 bis 13 hergestellt wird, zur Synthese einer Nukleinsäure.

**14.** Verfahren zur Synthese einer Nukleinsäure, das die folgenden Schritte umfasst:

(1) Bereitstellen des Biochips nach einem der Ansprüche 1 bis 9 oder des Biochips, der durch Verwenden des Verfahrens nach einem der Ansprüche 10 bis 13 hergestellt wird, wobei der auf dem Biochip befindliche Code einer Sequenz der zu synthetisierenden Nukleinsäure entspricht;

(2) Bereitstellen von vier Reaktionsbehältern, die jeweils zum Hinzufügen von Desoxyribonukleotiden A, T, C und G oder Ribonukleotiden A, U, C und G sowie zum Entschützen, Verkappen, Oxidieren und Waschen in jedem der Reaktionsbehälter verwendet werden;

(3) Erkennen des Codes, der auf dem Biochip getragen wird, und Bestimmen des hinzuzufügenden Desoxyribonukleotids oder Ribonukleotids gemäß der Sequenz, die dem Code entspricht, und Sortieren der Biochips mit demselben hinzuzufügenden Desoxyribonukleotid oder Ribonukleotid in denselben Reaktionsbehälter;

(4) Hinzufügen eines Entschützungsreagens in den Reaktionsbehälter, Eintauchen des Biochips in den Reaktionsbehälter zum Entschützen und Entleeren des Entschützungsreagens nach Abschluss der Reaktion;

(5) Hinzufügen eines Kopplungsreagens des entsprechenden Desoxyribonukleotids oder Ribonukleotids in den Reaktionsbehälter, Eintauchen des Biochips in den Reaktionsbehälter für die Kopplungsreaktion und Entleeren des Kopplungsreagens nach Abschluss der Reaktion;

(6) Hinzufügen eines Verkappungsreagens in den Reaktionsbehälter, Eintauchen des Biochips in den Reaktionsbehälter für die Verkappungsungsreaktion und Entleeren des Verkappungsreagens nach Abschluss der Reaktion;

(7) Hinzufügen eines Oxidationsreagens in den Reaktionsbehälter, Eintauchen des Biochips in den Reaktionsbehälter für die Oxidationsreaktion und Ablassen des Oxidationsreagens, nachdem die Reaktion abgeschlossen ist, wodurch das Hinzufügen eines Nukleotids abgeschlossen ist;

(8) gemäß der Sequenz der zu synthetisierenden Nukleinsäure, die dem Code entspricht, Wiederholen der Schritte (3) bis (7) einmal oder mehrmals, wodurch eine Nukleinsäure auf dem Biochip erzeugt wird, die eine vorbestimmte Sequenz aufweist; optional wobei das Verfahren ferner die folgenden Schritte umfasst:

(9) Schneiden der Nukleinsäure vom Biochip, wodurch die Nukleinsäure gewonnen wird;

**15.** Verfahren nach Anspruch 14, wobei vor der Nukleinsäuresynthese und nachdem der Biochip in den Reaktionsbehälter sortiert wurde, das Verkappungsreagenz in den Reaktionsbehälter hinzugefügt wird und der Biochip für die Verkappungsreaktion in den Reaktionsbehälter eingetaucht wird;
vorzugsweise wird zur Durchführung der Synthesereaktion eine Vielzahl von Chips gleichzeitig verwendet.

## Revendications

1. Biopuce portant une entité chimique et un code, dans laquelle le code présente une relation de correspondance unique avec la biopuce ; dans laquelle la biopuce présente une surface traitée avec un réactif de silanisation, et le réactif de silanisation est un réactif de silanisation à 50 % ; et dans laquelle l'entité chimique est une molécule de liaison capable d'initier une réaction de synthèse d'ADN/ARN, et la biopuce est constituée d'une plaquette de silicium.

2. Biopuce selon la revendication 1, dans laquelle
l'entité chimique est une molécule de liaison dotée d'un groupe fonctionnel réagissant avec un groupe amino sur une extrémité initiale et présentant un hydroxyle protégé par un groupe protecteur labile à l'acide sur une extrémité terminale, de préférence une molécule de liaison universelle.

3. Biopuce selon l'une des revendications 1 à 2, dans laquelle le code est une caractérisation spécifique de l'identité de la biopuce, comprenant un nombre, un symbole, un graphique et/ou un code d'identification ; de préférence, le code est un code-barres bidimensionnel.

4. Biopuce selon l'une quelconque des revendications 1 à 3, dans laquelle
la biopuce est choisie parmi une puce à code-barres bidimensionnelle non polie monoface, une puce à code-barres bidimensionnelle non polie à deux faces, une puce à code-barres bidimensionnelle simple polie sur une seule face, une puce à code-barres bidimensionnelle simple polie sur deux faces, une puce à code-barres bidimensionnelle simple polie sur deux faces et une puce à code-barres bidimensionnelle double polie sur deux faces.

5. Biopuce selon l'une quelconque des revendications 1 à 3, dans laquelle la biopuce présente une taille inférieure à 2 mm*2 mm, de préférence 0,5 mm*0,5 mm.

6. Biopuce selon l'une quelconque des revendications 1 à 3, dans laquelle la biopuce est faite de verre poreux, et le verre poreux présente une taille de particule de 5 µm à 2000 µm ; le verre poreux présente un diamètre de pore de 200 Ǻ à 5 000 Ǻ.

7. Biopuce selon l'une quelconque des revendications 1 à 3, dans laquelle la biopuce est choisie parmi une plaquette d'oxyde de silicium de 100 nm, une plaquette d'oxyde de silicium de 300 nm, une plaquette de quartz dépoli ou une plaquette de quartz transparent.

8. Biopuce selon l'une quelconque des revendications 1 à 3, dans laquelle la biopuce est une puce en quartz transparent.

9. Biopuce selon l'une quelconque des revendications 1 à 3, dans laquelle le réactif de silanisation est choisi parmi l'APTMS ou l'APTES.

10. Procédé de préparation de la biopuce selon l'une des revendications 1 à 9, comprenant les étapes suivantes :

    1) le codage de la biopuce ;
    2) le prétraitement de la biopuce ;
    3) la réalisation d'un traitement de silanisation sur la surface de la biopuce ; dans lequel le traitement de silanisation est effectué sur la surface de la biopuce en trempant la biopuce dans un réactif de silanisation et en effectuant un traitement aux ultrasons, et le réactif de silanisation est un réactif de silanisation à 50 % ;
    4) la modification chimique de la surface de la biopuce ; et
    5) éventuellement, la détection ou l'analyse quantitative de la modification chimique ;

    de préférence, le prétraitement comprend un traitement acide, un traitement alcalin, un traitement par ultrasons, un nettoyage au plasma, un lavage à l'acétone ou une combinaison de ceux-ci, dans lequel l'acide est choisi parmi l'acide sulfurique, l'acide chlorhydrique, l'acide phosphorique et le liquide de piranha, l'alcalin est choisi parmi l'hydroxyde de sodium et l'hydroxyde de potassium ; de préférence, le procédé de prétraitement est un traitement acide suivi d'un nettoyage au plasma ; plus préférentiellement, le procédé de prétraitement est un traitement à l'hydroxyde de sodium suivi d'un nettoyage au plasma et d'un lavage à l'acétone ;
    de préférence, la modification chimique de la surface de la biopuce est obtenue en liant l'entité chimique à la surface de la biopuce.

**11.** Procédé selon la revendication 10, dans lequel un code-barres bidimensionnel est imprimé sur la biopuce au moyen d'une impression laser, de préférence, le codage est effectué à l'aide d'un laser rouge dans un mode à code-barres bidimensionnel à points.

**12.** Procédé selon la revendication 10, dans lequel le réactif de silanisation comprend l'APTES et le PTES.

**13.** Utilisation de la biopuce selon l'une quelconque des revendications 1 à 9 ou de la biopuce préparée en utilisant le procédé selon l'une quelconque des revendications 10 à 13 dans la synthèse d'un acide nucléique.

**14.** Procédé de synthèse d'un acide nucléique comprenant les étapes consistant à :

(1) fournir la biopuce selon l'une quelconque des revendications 1 à 9 ou la biopuce préparée en utilisant le procédé selon l'une quelconque des revendications 10 à 13, dans lequel le code porté sur la biopuce correspond à une séquence de l'acide nucléique à synthétiser ;
(2) fournir quatre cuves de réaction utilisées respectivement pour l'addition des désoxyribonucléotides A, T, C et G ou de ribonucléotides A, U, C et G, ainsi que pour la déprotection, le capsulage, l'oxydation et le lavage dans chacune des cuves de réaction ;
(3) reconnaître le code porté par la biopuce, et déterminer le désoxyribonucléotide ou le ribonucléotide à ajouter en fonction de la séquence correspondant au code, et trier des biopuces contenant le même désoxyribonucléotide ou ribonucléotide à ajouter dans le même réservoir de réaction ;
(4) ajouter un réactif de déprotection dans le réservoir de réaction, tremper la biopuce dans le réservoir de réaction pour la déprotection, et évacuer le réactif de déprotection une fois la réaction terminée ;
(5) ajouter un réactif de couplage du désoxyribonucléotide ou du ribonucléotide correspondant dans le réservoir de réaction, tremper la biopuce dans le réservoir de réaction pour la réaction de couplage, et décharger le réactif de couplage une fois la réaction terminée ;
(6) ajouter un réactif de capsulage dans le réservoir de réaction, tremper la biopuce dans le réservoir de réaction pour la réaction de capsulage, et évacuer le réactif de capsulage une fois la réaction terminée ;
(7) ajouter un réactif oxydant dans le réservoir de réaction, tremper la biopuce dans le réservoir de réaction pour la réaction d'oxydation, et décharger le réactif oxydant une fois la réaction terminée, achevant ainsi l'ajout d'un nucléotide ;
(8) selon la séquence d'acide nucléique à synthétiser correspondant au code, répéter les étapes (3) à (7) une ou plusieurs fois, en générant ainsi un acide nucléique présentant une séquence prédéterminée sur la biopuce ; éventuellement, le procédé comprend en outre les étapes suivantes :
(9) couper l'acide nucléique de la biopuce, afin d'obtenir ainsi l'acide nucléique ;

**15.** Procédé selon la revendication 14, dans lequel, avant la synthèse de l'acide nucléique et après le tri de la biopuce dans le réservoir de réaction, le réactif de capsulage est ajouté dans le réservoir de réaction, et la biopuce est trempée dans le réservoir de réaction pour la réaction de bouchage ;
de préférence, plusieurs puces sont utilisées simultanément pour effectuer la réaction de synthèse.

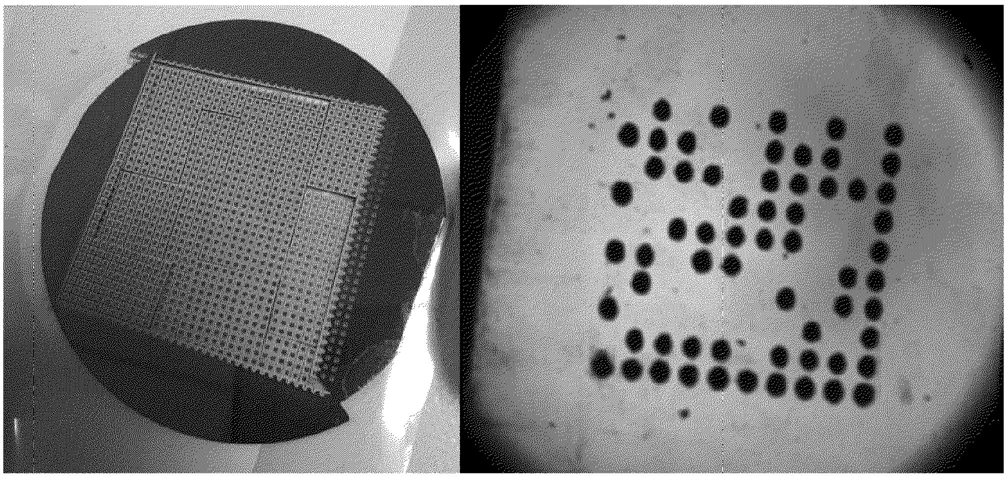

Fig. 1

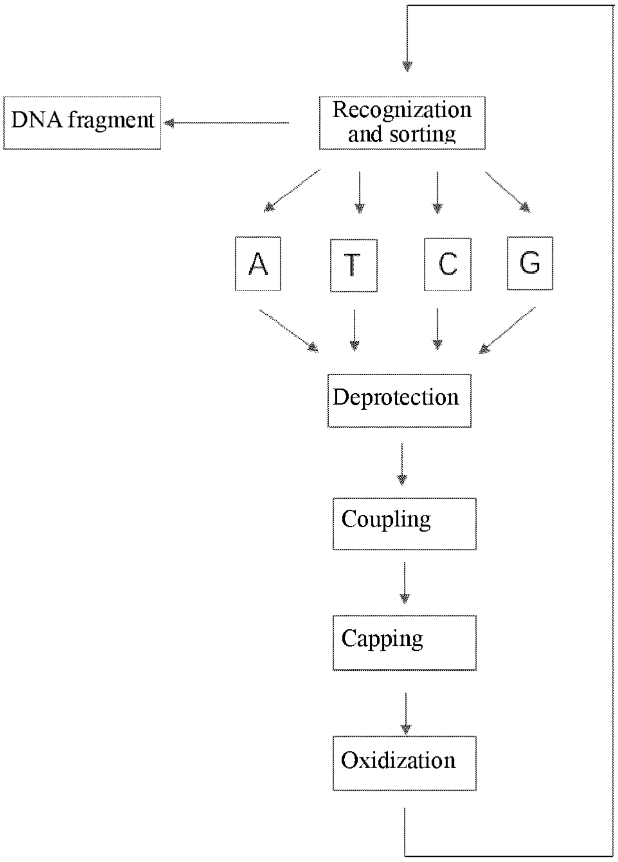
DNA fragment
Recognization and sorting
A
T
C
G
Deprotection
Coupling
Capping
Oxidization

Fig. 2

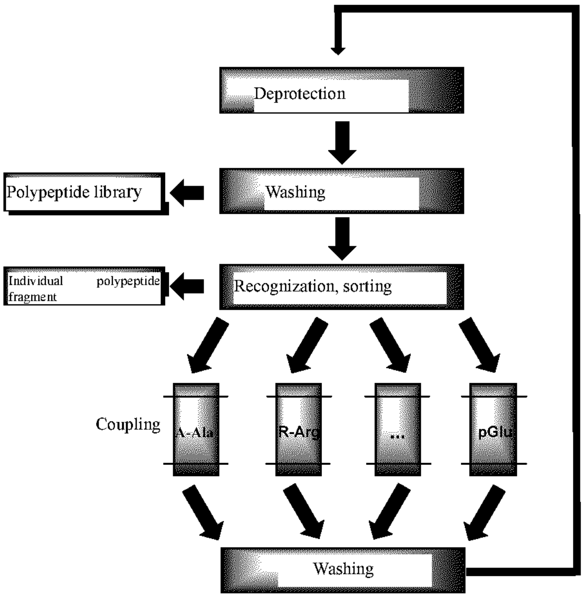
Deprotection
Polypeptide library
Washing
Individual polypeptide fragment
Recognization, sorting
Coupling
A-Ala
R-Arg
...
pGlu
Washing

Fig. 3

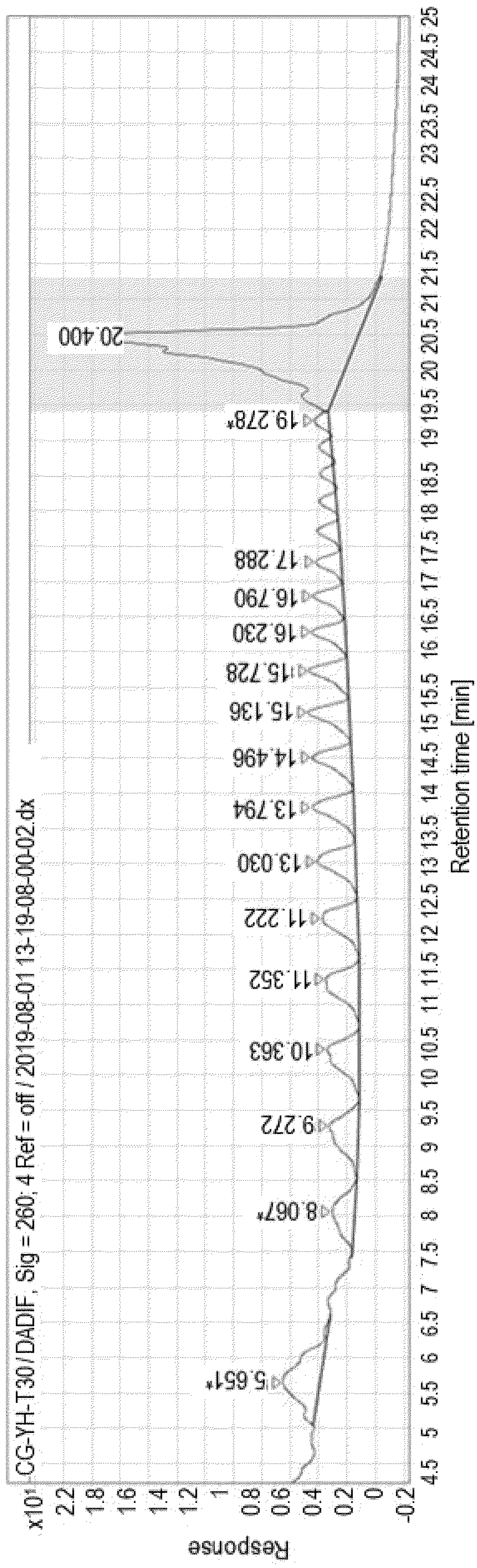
CG-YH-T30 / DADIF, Sig = 260; 4 Ref = off / 2019-08-01 13-19-08-00-02.dx
x10¹
Response
Retention time [min]
5.651*
8.067*
9.272
10.363
11.352
11.222
13.030
13.794
14.496
15.136
15.728
16.230
16.790
17.288
19.278*
20.400

Fig. 4A

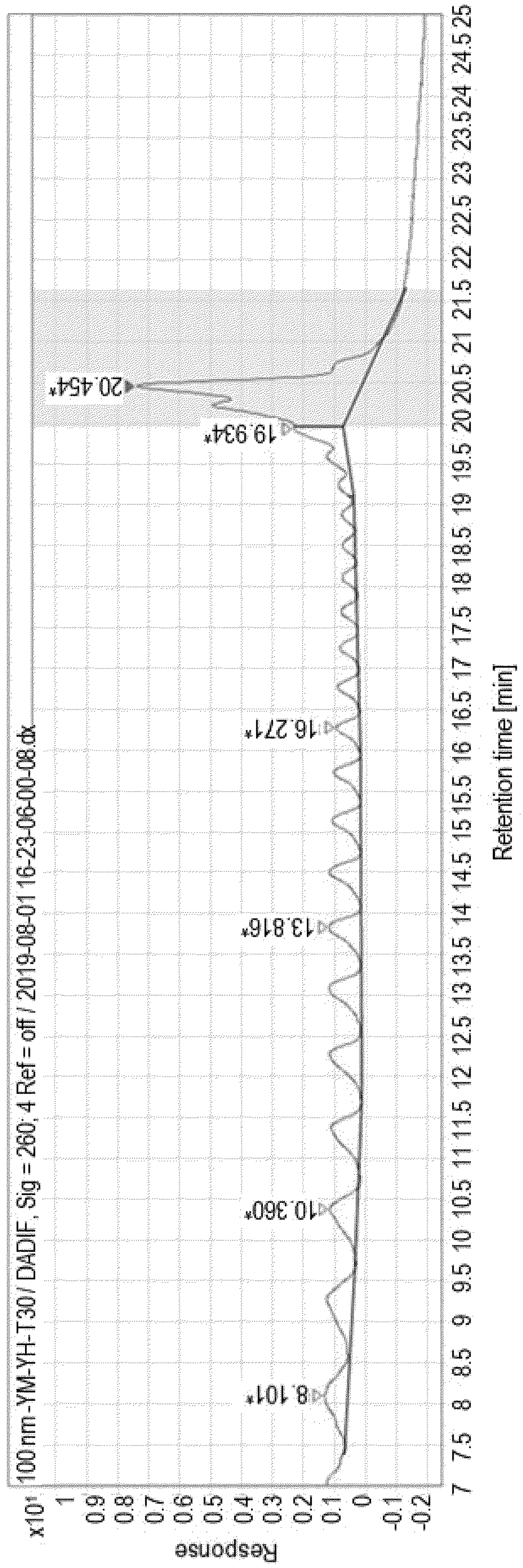

100nm-YM-YH-T30/ DADIF, Sig = 260, 4 Ref = off / 2019-08-01 16-23-06-00-08.dx
x10¹
Response
1
0.9
0.8
0.7
0.6
0.5
0.4
0.3
0.2
0.1
0
-0.1
-0.2
8.101*
10.360*
13.816*
16.271*
19.934*
20.454*
7 7.5 8 8.5 9 9.5 10 10.5 11 11.5 12 12.5 13 13.5 14 14.5 15 15.5 16 16.5 17 17.5 18 18.5 19 19.5 20 20.5 21 21.5 22 22.5 23 23.5 24 24.5 25
Retention time [min]


Fig. 4B

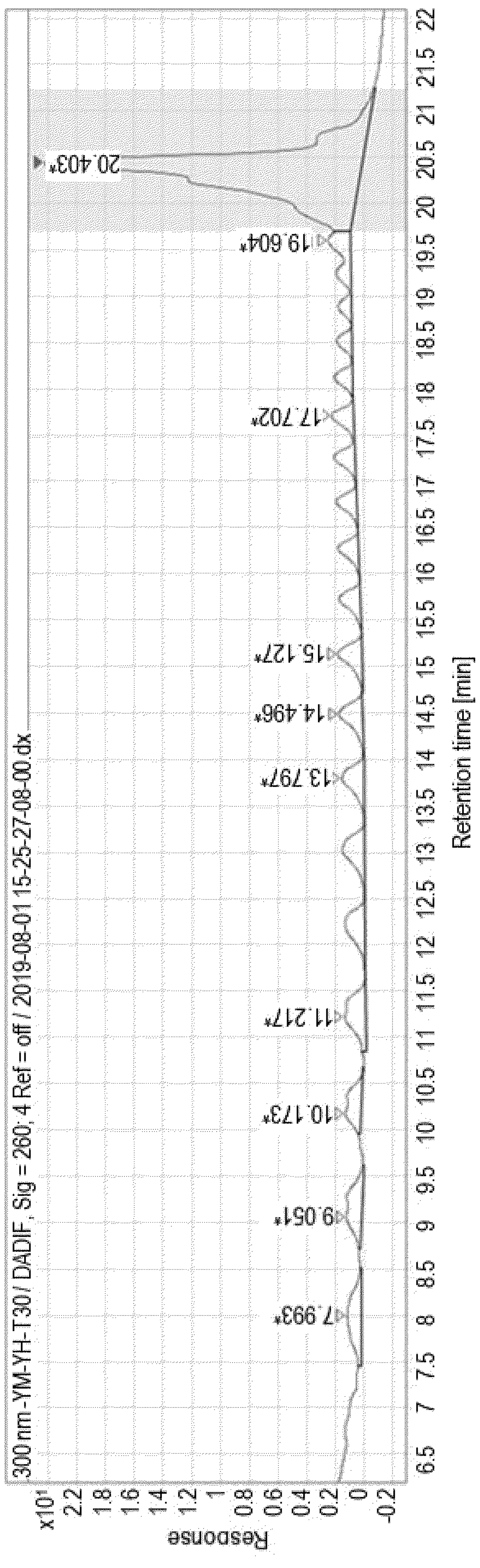
300 nm -YM-YH-T30 / DADIF, Sig = 260; 4 Ref = off / 2019-08-01 15-25-27-08-00.dx
Response
x10¹
2.2
1.8
1.6
1.4
1.2
1
0.8
0.6
0.4
0.2
0
-0.2
Retention time [min]
6.5
7
7.5
8
8.5
9
9.5
10
10.5
11
11.5
12
12.5
13
13.5
14
14.5
15
15.5
16
16.5
17
17.5
18
18.5
19
19.5
20
20.5
21
21.5
22
7.993*
9.051*
10.173*
11.217*
13.797*
14.496*
15.127*
17.702*
19.604*
20.403*

Fig. 4C

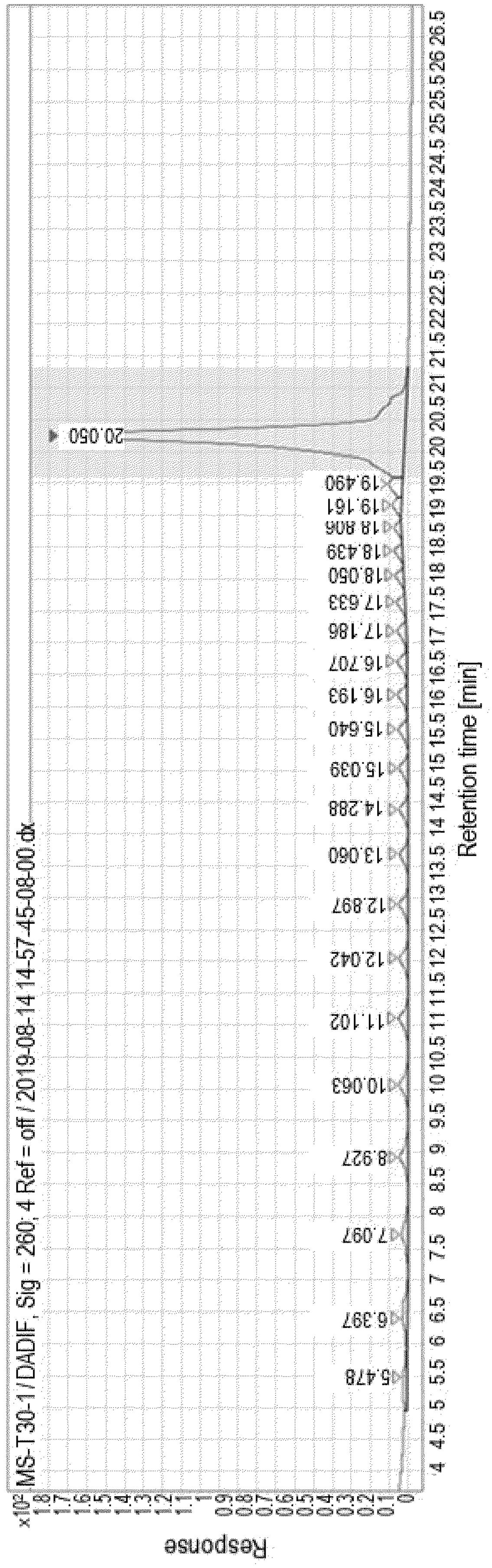
MS-T30-1 / DADIF, Sig = 260; 4 Ref = off / 2019-08-14 14-57-45-08-00.dx
Response
x10^2
Retention time [min]
20.050
19.490
19.161
18.806
18.439
18.050
17.633
17.186
16.707
16.193
15.640
15.039
14.288
13.060
12.897
12.042
11.102
10.063
8.927
7.097
6.397
5.478

Fig. 4D

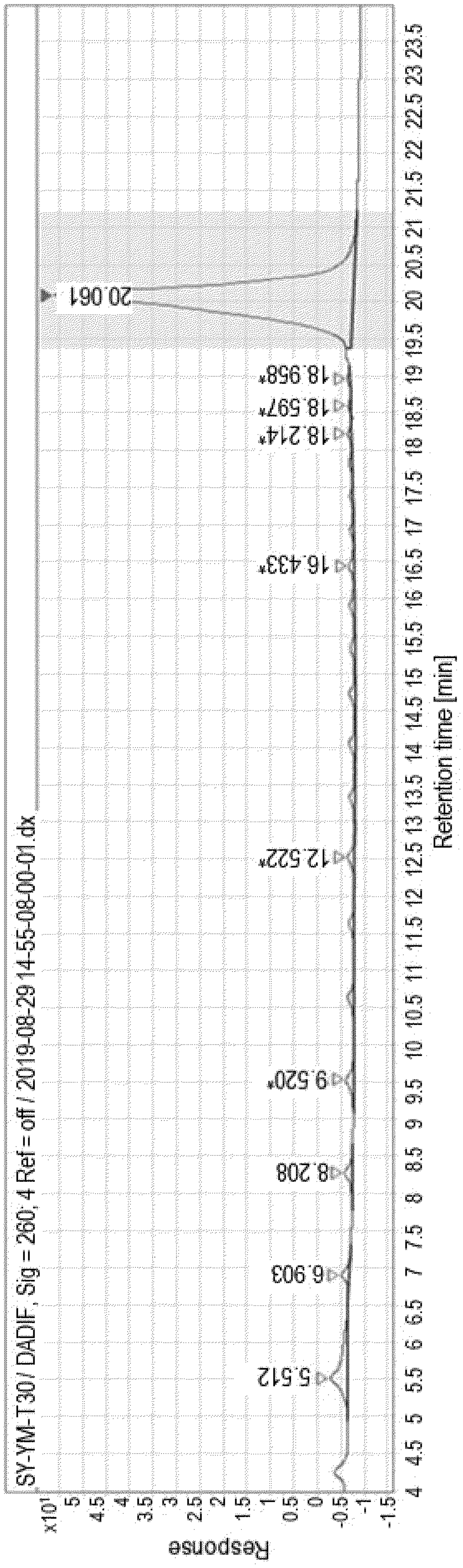
SY-YM-T30 / DADIF, Sig = 260; 4 Ref = off / 2019-08-29 14-55-08-00-01.dx
x10[1]
Response
Retention time [min]
20.061
18.958*
18.597*
18.214*
16.433*
12.522*
9.520*
8.208
6.903
5.512

Fig. 4E

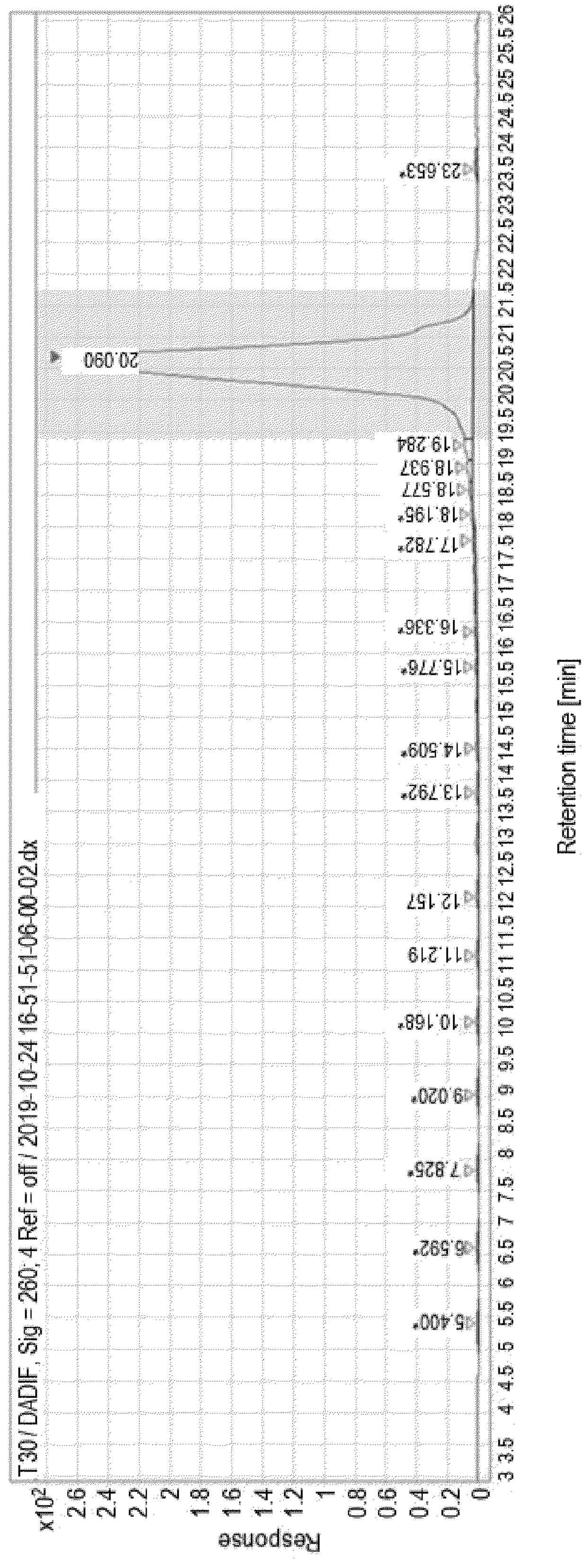
T30 / DADIF, Sig = 260; 4 Ref = off / 2019-10-24 16-51-51-06-00-02.dx
x10²
Response
20.090
19.284
18.937
18.577
18.195*
17.782*
16.336*
15.776*
14.509*
13.792*
12.157
11.219
10.168*
9.020*
7.825*
6.592*
5.400*
23.653*
Retention time [min]

Fig. 4F

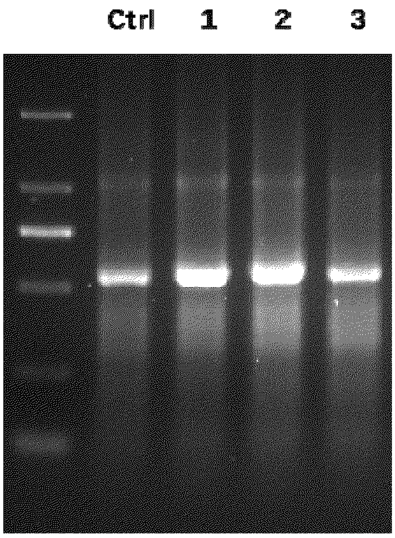


Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2007218484 A
- US 2004152081 A
- US 2003203369 A


### Non-patent literature cited in the description


- **MICHELWN, A. M.** ; **TODD, A. R.** *J. Chem. Soc.*, 1955
- **GILHAM, P. T.** ; **KHORANA, H. G.** *J. A. m. Cliem. Soc.*, 1958